# EUROPÄISCHE PATENTSCHRIFT

(11) **EP 2 729 472 B1**
(45) Veröffentlichungstag und Bekanntmachung des Hinweises auf die Patenterteilung: **24.08.2016**
(21) Anmeldenummer: 12733130.4
(22) Anmeldetag: 06.07.2012
(51) Int. Cl.: C07D 498/04, A61K 31/424, A61P 17/02

(54) **CARBONSÄUREDERIVATE MIT EINEM OXAZOLO[4,5-C]PYRIDINRING**
CARBOXYLIC ACID DERIVATIVES WITH AN OXAZOLO[4,5-C]PYRIDINE RING
DÉRIVÉ D'ACIDE CARBONIQUE DOTÉ D'UN ANNEAU D'OXAZOLO[4,5-C]-PYRIDINE

(30) Priorität: 07.07.2011 EP 11305876; 11.05.2012 EP 12305524
(43) Veröffentlichungstag der Anmeldung: 14.05.2014
(73) Patentinhaber: SANOFI, 75008 Paris (FR)
(72) Erfinder: KADEREIT, Dieter, 65926 Frankfurt am Main (DE); SCHÄFER, Matthias, 65926 Frankfurt am Main (DE); HACHTEL, Stephanie, 65926 Frankfurt am Main (DE); HUEBSCHLE, Thomas, 65926 Frankfurt am Main (DE); HISS, Katrin, 65926 Frankfurt am Main (DE)
(74) Vertreter: Michalski Hüttermann & Partner Patentanwälte mbB
(86) Internationale Anmeldenummer: PCT/EP2012/063294
(87) Internationale Veröffentlichungsnummer: WO 2013/004824

(56) Entgegenhaltungen:
- WO-A1-2009/154775
- WO-A1-2010/006704
- WO-A2-2009/155017

## Beschreibung

Die Erfindung betrifft Carbonsäurederivate mit einem Oxazolo[4,5-c]pyridinring und deren physiologisch akzeptable Salze.

Es sind bereits strukturähnliche Verbindungen im Stand der Technik beschrieben (siehe WO2009/154775), die zur Behandlung multipler Sklerose geeignet sind. Die Wirkungsweise dieser Verbindungen besteht darin, durch Aktivierung des EDG-1 Rezeptors eine Desensitisierung des EDG-1 Signalweges zu verursachen (sog. Superagonismus), der dann einem funktionellen Antagonismus des EDG-1-Signalweges gleichkommt. Systemisch bedeutet das, daß vor allem auf Lymphozyten der EDG-1 Signalweg dauerhaft unterdrückt wird, wodurch diese Zellen nicht mehr chemotaktisch dem S1 P Gradienten zwischen Blut und Lymphflüssigkeit folgen können. Dies bedingt, dass die betroffenen Lymphozyten nicht mehr das sekundäre lymphatische Gewebe verlassen können (verstärktes Homeing) und die Zahl der frei zirkulierenden Lymphozyten im Plasma stark gesenkt wird. Dieser Mangel an Lymphozyten im Plasma (Lymphopenie) bewirkt eine Immunsuppression, die zwingend für den Wirkmechanismus der in WO 2009/154775 beschriebenen EDG-1-Rezeptor-Modulatoren erforderlich ist.

Der Erfindung lag die Aufgabe zugrunde, Verbindungen zur Verfügung zu stellen, die eine therapeutisch verwertbare Wirkung entfalten. Insbesondere bestand die Aufgabe darin, neue Verbindungen zu finden, die spezifisch zur Wundheilung und insbesondere zur Behandlung von Wundheilungsstörungen von Diabetes Patienten geeignet sind. Weiter war es wünschenswert Verbindungen zur Verfügung zustellen, die zur Behandlung des Diabetische Fußsyndroms (DFS) geeignet sind.

Weiter war es wünschenswert eine wiederholbare Aktivierung des EDG-1-Rezeptor Signalweges zu erreichen, was damit pharmakologisch eine persistente Aktivierung des EDG-1 Signalweges ermöglicht.

Die Erfindung betrifft daher Verbindungen der Formel I 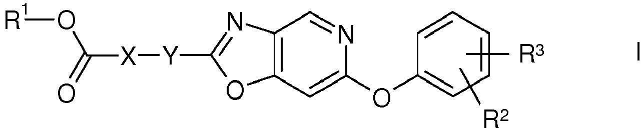 worin X, Y, R¹, R² und R³ wie nachstehend definiert sind.

Der Wirkmechanismus der Verbindungen der Formel I beruht nicht auf Desensitisierung des EDG-1-Signalweges und steht somit dem in WO 2009/154775 beschriebenen Wirkmechanismus diametral gegenüber. Die Erfindung betrifft ferner Verfahren zur Herstellung von Verbindungen der Formel I, ihre Verwendung, insbesondere als Wirkstoff in Pharmazeutika, und pharmazeutische Zusammensetzungen, die sie enthalten.
Patienten mit Diabetes haben gegenüber gesunden Menschen eine verzögerte Wundheilung und eine erhöhte Infektionsrate, vor allem bei länger währender Hyperglykämie, zum Beispiel hervorgerufen durch eine schlechte Blutzuckereinstellung. Zu den Ursachen gehören Durchblutungsstörungen, vor allem im Bereich der kleinen Gefäße, die zu einer verschlechterten Sauerstoff- und Nährstoffversorgung des Gewebes führen. Außerdem besteht eine verminderte Zellteilungs- und Zellmigrationsrate von Keratinozyten, Fibroblasten und dermalen Endothelzellen. Zusätzlich ist die Aktivität verschiedener Abwehrzellen (Granulozyten) mit reduzierter Phagozytose (Aufnahme und Zerstörung von Bakterien) eingeschränkt. Auch die Funktion der Antikörper (Immunglobuline) gegen Bakterien ist bei hohen Blutzuckerwerten eingeschränkt. Dementsprechend müssen Wunden und Infektionen bei Diabetes-Patienten besonders versorgt werden.

Der Edg-1-Rezeptor gehört zur Familie der Edg-Rezeptoren (Edg = Endothelial Differentiation Gene) von gegenwärtig acht identifizierten GPCRs (G-Protein-gekoppelten Rezeptoren) der Klasse A. Diese Familie kann in Unterfamilien von durch Sphingosin-1-Phosphat (S1 P) aktivierten Rezeptoren (fünf Mitglieder) und durch Lysophosphatidsäure (LPA) aktivierte Rezeptoren (drei Mitglieder) unterteilt werden. Der endogene Ligand S1 P ist ein pluripotentes Lysophospholipid, das durch Aktivierung von GPCRs aus der Edg-Rezeptorfamilie, nämlich Edg-1 (= S1 P1), Edg-3 (= S1 P3), Edg-5 (= S1 P2), Edg-6 (= S1 P4) und Edg-8 (S1 P5), auf verschiedene Zelltypen wirkt. Wenngleich S1 P auch als intrazellulärer Botenstoff beschrieben wird, werden zahlreiche zelluläre Antworten von S1 P über die Aktivierung von Edg-Rezeptoren - vermittelt werden. S1 P wird durch die Enzymfamilie der Sphingosinkinasen (SPHK) erzeugt und durch verschiedene Phosphatasen oder Lyasen abgebaut.
Bekannte Indikationen von Edg-1-Rezeptor-Agonisten sind beispielsweise Herz-Kreislauf-Erkrankungen, Atherosklerose, Herzversagen, Cardioprotektion, periphere arterielle Verschlußkrankheit, Nierenerkrankungen und Atemwegserkrankungen.

Gegenstand der vorliegenden Erfindung sind Verbindungen der Formel I in einer beliebigen ihrer stereoisomeren Formen oder einer Mischung von stereoisomeren Formen in beliebigem Verhältnis oder ein physiologisch akzeptables Salz davon oder ein physiologisch akzeptables Solvat einer derartigen Verbindung oder eines derartigen Salzes, 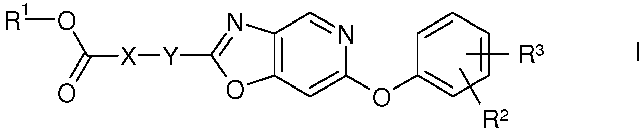 worin
X ausgewählt ist aus (C₁-C₆)-Alkandiyloxy, wobei das Sauerstoffatom der (C₁-C₆)-Alkandiyloxygruppe an die Gruppe Y gebunden ist;
Y Phenylen ist, wobei das Phenylen gegebenenfalls an einem oder mehreren Ringkohlenstoffatomen durch gleiche oder verschiedene Substituenten R⁵ substituiert ist;
R¹ ausgewählt ist aus Wasserstoff und (C₁-C₄)-Alkyl;
R² und R³ unabhängig voneinander ausgewählt sind aus H, Halogen, Hydroxy, (C₁-C₄)-Alkyl-, (C₁-C₄)-Alkyloxy, (C₁-C₄)-Alkyl-S(O)ₘ-, Amino, Nitro, Cyano, Hydroxycarbonyl, (C₁-C₄)-Alkyloxycarbonyl, Aminocarbonyl und Aminosulfonyl, (C₃-C₇)-Cycloalkyl-C_{w}H_{2w}- und Oxy, wobei w aus 0, 1 und 2 ausgewählt ist;
R⁵ (C₁-C₄)-Alkyl ist;
m aus 0, 1 und 2 ausgewählt ist.

Strukturelemente wie Gruppen, Substituenten, Heteroringglieder, Zahlen oder andere Merkmale, beispielsweise Alkylgruppen, Gruppen wie R⁵, Zahlen wie m, die in den Verbindungen der Formel I mehrmals vorkommen können, können alle unabhängig voneinander eine beliebige der angegebenen Bedeutungen besitzen und in jedem Fall gleich oder voneinander verschieden sein. Beispielsweise können die Alkylgruppen in einer Dialkylaminogruppe gleich oder verschieden sein.

Alkylgruppen können linear, d.h. geradkettig, oder verzweigt sein. Dies gilt auch, wenn sie Teil anderer Gruppen, beispielsweise Alkyloxygruppen (= Alkoxygruppen, Alkyl-O-Gruppen), Alkyloxycarbonylgruppen oder alkylsubstituierter Aminogruppen, sind oder wenn sie substituiert sind. Je nach der jeweiligen Definition kann die Zahl der Kohlenstoffatome in einer Alkylgruppe 1, 2, 3, 4, 5 oder 6 oder 1, 2, 3 oder 4 oder 1, 2 oder 3 betragen. Beispiele für Alkyl sind Methyl, Ethyl, Propyl einschließlich n-Propyl und Isopropyl, Butyl, einschließlich n-Butyl, sek.-Butyl, Isobutyl und tert.-Butyl, Pentyl einschließlich n-Pentyl, 1-Methylbutyl, Isopentyl, Neopentyl und tert.-Pentyl und Hexyl einschließlich n-Hexyl, 3,3-Dimethylbutyl und Isohexyl. Substituierte Alkylgruppen, können in beliebigen Positionen substituiert sein, mit der Maßgabe, daß die jeweilige Verbindung ausreichend stabil und für den gewünschten Zweck wie die Verwendung als Arzneistoff geeignet ist. Die Voraussetzung, daß eine spezifische Gruppe und eine Verbindung der Formel I ausreichend stabil und für den gewünschten Zweck wie die Verwendung als Arzneistoff geeignet sind, gilt allgemein in bezug auf die Definitionen aller Gruppen in den Verbindungen der Formel I.

Soweit anwendbar, gelten die vorstehenden Erklärungen bezüglich Alkylgruppen entsprechend für zweiwertige Alkylgruppen wie die Gruppen Alkandiyl CᵤH₂ᵤ, CᵥH₂ᵥ, C_{w}H_{2w} und C_{z}H_{2z}, die somit ebenfalls linear und verzweigt sein können.

Die Zahl der Ringkohlenstoffatome in einer Cycloalkylgruppe kann 3, 4, 5, 6 oder 7 betragen. In einer Ausführungsform der Erfindung beträgt die Zahl der Ringkohlenstoffatome in einer Cycloalkylgruppe unabhängig von der Zahl der Ringkohlenstoffatome in einer anderen Cycloalkylgruppe 3, 4, 5 oder 6, in einer anderen Ausführungsform 3, 4 oder 5, in einer anderen Ausführungsform 3 oder 4, in einer anderen Ausführungsform 3, in einer anderen Ausführungsform 5, 6 oder 7, in einer anderen Ausführungsform 5 oder 6, in einer anderen Ausführungsform 6 oder 7, in einer anderen Ausführungsform 6. Dies gilt entsprechend für zweiwertige Cycloalkylgruppen, d.h. Cycloalkandiylgruppen, die über ein oder zwei beliebige Ringkohlenstoffatome an die benachbarten Gruppen gebunden sein können. Beispiele für Cycloalkylgruppen sind Cyclopropyl, Cyclobutyl, Cyclopentyl, Cyclohexyl und Cycloheptyl. Beispiele für zweiwertige Cycloalkylgruppen sind Cyclopropan-1,1-diyl, Cyclopropan-1,2-diyl, Cyclobutan-1,3-diyl, Cyclopentan-1,1-diyl, Cyclopentan-1,2-diyl, Cyclopentan-1,3-diyl, Cyclohexan-1,1-diyl, Cyclohexan-1,2-diyl, Cyclohexan-1,3-diyl, Cyclohexan-1,4-diyl, Cycloheptan-1,4-diyl. Unabhängig voneinander und unabhängig von anderen Substituenten sind Cycloalkylgruppen und Cycloalkandiylgruppen gegebenenfalls durch einen oder mehrere gleiche oder verschiedene (C₁-C₄)-Alkylsubstituenten substituiert, die in beliebigen Positionen stehen können, d.h. Cycloalkylgruppen können durch Alkylsubstituenten unsubstituiert oder durch Alkylsubstituenten, beispielsweise 1, 2, 3 oder 4 oder 1 oder 2 (C₁-C₄)-Alkylsubstituenten, beispielsweise Methylgruppen, substituiert sein. Beispiele für alkylsubstituierte Cycloalkylgruppen und Cycloalkandiylgruppen sind 4-Methylcyclohexyl, 4-tert.-Butylcyclohexyl oder 2,3-Dimethylcyclopentyl, 2,2-Dimethylcyclopropan-1,1-diyl, 2,2-Dimethylcyclopropan-1,2-diyl, 2,2-Dimethylcyclopentan-1,3-diyl, 6,6-Dimethylcycloheptan-1,4-diyl. Beispiele für Cycloalkylalkylgruppen, die beispielsweise Gruppen wie (C₃-C₅)-Cycloalkyl-C_{z}H_{2z}-repräsentieren können, sind Cyclopropylmethyl, Cyclobutylmethyl, Cyclopentylmethyl, Cyclohexylmethyl, Cycloheptylmethyl, 1-Cyclopropylethyl, 2-Cyclopropylethyl, 1-Cyclobutylethyl, 2-Cyclobutylethyl, 2-Cyclopentylethyl, 2-Cyclohexylethyl, 2-Cycloheptylethyl.

Unabhängig voneinander und unabhängig von anderen Substituenten können Alkylgruppen, zweiwertige Alkylgruppen, Cycloalkylgruppen und zweiwertige Cycloalkylgruppen gegebenenfalls durch einen oder mehrere Fluorsubstituenten substituiert sein, die in beliebigen Positionen stehen können, d.h. diese Gruppen können durch Fluorsubstituenten unsubstituiert oder durch Fluorsubstituenten, beispielsweise 1, 2, 3, 4, 5, 6, 7, 8, 9, 10, 11, 12 oder 13 oder 1, 2, 3, 4, 5, 6, 7, 8 oder 9 oder 1, 2, 3, 4, 5, 6 oder 7 oder 1, 2, 3, 4 oder 5 oder 1, 2 oder 3 oder 1 oder 2 Fluorsubstituenten, substituiert sein. Beispiele für fluorsubstituierte derartige Gruppen sind Trifluormethyl, 2-Fluorethyl, 2,2,2-Trifluorethyl, Pentafluorethyl, 3,3,3-Trifluorpropyl, 2,2,3,3,3-Pentafluorpropyl, 4,4,4-Trifluorbutyl, Heptafluorisopropyl, -CHF-, -CF₂-, -CF₂-CH₂-, -CH₂-CF₂-, -CF₂-CF₂-, -CF(CH₃)-, -C(CF₃)₂-, 1-Fluorcyclopropyl, 2,2-Difluorcyclopropyl, 3,3-Difluorcyclobutyl, 1-Fluorcyclohexyl, 4,4-Difluorcyclohexyl, 3,3,4,4,5,5-Hexafluorcyclohexyl, 2,2-Difluorcyclopropan-1,2-diyl. Beispiele für Alkyloxygruppen, in denen die Alkylgruppierung fluorsubstituiert ist, sind Trifluormethoxy, 2,2,2-Trifluorethoxy, Pentafluorethoxy und 3,3,3-Trifluorpropoxy. In einer Ausführungsform der Erfindung beträgt die Gesamtzahl der Fluorsubstituenten und (C₁-C₄)-Alkylsubstituenten, die unabhängig von anderen Substituenten gegebenenfalls an Cycloalkylgruppen und Cycloalkandiylgruppen in den Verbindungen der Formel I vorliegen, 1, 2, 3, 4, 5, 6, 7, 8, 9, 10 oder 11, in einer anderen Ausführungsform 1, 2, 3, 4, 5, 6, 7, 8 oder 9, in einer anderen Ausführungsform 1, 2, 3, 4 oder 5, in einer anderen Ausführungsform 1, 2, 3 oder 4.

Gruppen wie Phenyl, die gegebenenfalls durch einen oder mehrere Substituenten substituiert sind, können unsubstituiert oder substituiert sein, beispielsweise durch 1, 2, 3, 4 oder 5 oder 1, 2, 3 oder 4 oder 1, 2 oder 3 oder 1 oder 2 oder 1 gleiche oder verschiedene Substituenten, die in beliebigen Positionen stehen können. In einer Ausführungsform der Erfindung ist die Gesamtzahl der Nitrosubstituenten in einer Verbindung der Formel I nicht größer als zwei.

Wenn eine Oxogruppe an ein Kohlenstoffatom gebunden ist, ersetzt sie zwei Wasserstoffatome an einem Kohlenstoffatom des zugrundeliegenden Systems. Somit wird eine CH₂-Gruppe in einer Kette oder einem Ring dann, wenn sie durch Oxo, d.h. durch ein doppelt gebundenes Sauerstoffatom, substituiert ist, zu einer C(O)-Gruppe (= C(=O)-Gruppe). Offensichtlich kann eine Oxogruppe nicht als Substituent an einem Kohlenstoffatom in einem aromatischen Ring wie beispielsweise in einer Phenylgruppe vorkommen. Wenn ein Ringschwefelatom in einer heterocyclischen Gruppe eine oder zwei Oxogruppen tragen kann, handelt es sich in dem Fall, daß es keine Oxogruppe trägt, um ein nichtoxidiertes Schwefelatom S, oder in dem Fall, daß es eine Oxogruppe trägt, um eine S(O)-Gruppe (Sulfoxidgruppe, S-Oxid-Gruppe) oder in dem Fall, daß es zwei Oxogruppen trägt, um eine S(O)₂-Gruppe (= Sulfongruppe, S,S-Dioxid-Gruppe).

Die vorliegende Erfindung schließt alle stereoisomeren Formen der Verbindungen der Formel I und ihre Salze und Solvate ein. Bezüglich jedes chiralen Zentrums können die Verbindungen der Formel I unabhängig von jedem anderen chiralen Zentrum in S-Konfiguration oder weitgehend S-Konfiguration oder in R-Konfiguration oder weitgehend R-Konfiguration oder als Mischung des S-Isomers und des R-Isomers in beliebigen Verhältnissen vorliegen. Die Erfindung schließt alle möglichen Enantiomere und Diastereomere und Mischungen von zwei oder mehr Stereoisomeren, zum Beispiel Gemische von Enantiomeren und/oder Diastereomeren, in allen Verhältnissen ein. Somit können erfindungsgemäße Verbindungen, die als Enantiomere existieren können, in enantiomerenreiner Form sowohl als linksdrehende als auch rechtsdrehende Antipoden und in Form von Mischungen der beiden Enantiomere in allen Verhältnissen einschließlich Racematen vorliegen. Im Fall einer E/Z-Isomerie bzw. cis/trans-Isomerie, beispielsweise an Doppelbindungen oder Ringen wie Cycloalkylringen, schließt die Erfindung sowohl die E-Form als auch die Z-Form bzw. die cis-Form und die trans-Form sowie Mischungen dieser Formen in allen Verhältnissen ein. In einer Ausführungsform der Erfindung handelt es sich bei einer Verbindung, die in zwei oder mehr stereoisomeren Formen vorkommen kann, um ein reines oder weitgehend reines einzelnes Stereoisomer. Die Herstellung von einzelnen Stereoisomeren kann beispielsweise durch Trennung einer Mischung von Isomeren nach üblichen Methoden, beispielsweise durch Chromatographie oder Kristallisation, durch Verwendung von stereochemisch einheitlichen Ausgangsstoffen bei der Synthese oder durch stereoselektive Synthese erfolgen. Gegebenenfalls kann vor einer Trennung von Stereoisomeren eine Derivatisierung durchgeführt werden. Die Trennung einer Mischung von Stereoisomeren kann auf der Stufe der Verbindung der Formel I oder auf der Stufe eines Ausgangsstoffs oder eines Zwischenprodukts im Verlauf der Synthese durchgeführt werden. Die vorliegende Erfindung schließt auch alle tautomeren Formen der Verbindungen der Formel I und ihre Salze und Solvate ein.

Wenn die Verbindungen der Formel I eine oder mehrere saure und/oder basische Gruppen, d.h. salzbildende Gruppen, enthalten, schließt die Erfindung auch ihre entsprechenden physiologisch oder toxikologisch akzeptablen Salze, d.h. nichttoxischen Salze, insbesondere ihre pharmazeutisch akzeptablen Salze, ein.

Die vorliegende Erfindung schließt alle Solvate von Verbindungen der Formel I, beispielsweise Hydrate oder Addukte mit Alkoholen wie (C₁-C₄)-Alkanolen, aktive Metaboliten der Verbindungen der Formel I und auch Prodrugs und Derivate der Verbindungen der Formel I, die in vitro nicht unbedingt pharmakologische Wirkung zeigen, aber in vivo in pharmakologisch wirksame Verbindungen umgewandelt werden, beispielsweise Ester oder Amide von Carbonsäuregruppen, ein.

Die Alkandiylgruppen, die in der Gruppe X vorkommen, können linear oder verzweigt sein, wie bereits bezüglich derartiger Gruppen im allgemeinen angegeben, und diese Gruppen können über beliebige Positionen an die benachbarten Gruppen, d.h. die Gruppe R¹O-C(O) und die Gruppe Y oder im Fall der Gruppe Alkandiyloxy an das Sauerstoffatom der Alkandiyloxygruppe, gebunden sein. Die benachbarten Gruppen können an das gleiche Kohlenstoffatom oder verschiedene Kohlenstoffatome in der Gruppe X gebunden sein. In einer Ausführungsform besteht die Kette von Kohlenstoffatomen in einer Alkandiylgruppe, die in der Gruppe X vorkommt, die die Gruppe R¹O-C(O) direkt mit der Gruppe Y oder im Fall der Gruppe Alkandiyloxy mit dem Sauerstoffatom der Alkandiyloxygruppe verbindet, aus 1, 2, 3 oder 4 Kohlenstoffatomen, in einer anderen Ausführungsform aus 1, 2 oder 3 Kohlenstoffatomen, in einer anderen Ausführungsform aus 1 oder 2 Kohlenstoffatomen, in einer anderen Ausführungsform aus 1 Kohlenstoffatom. In einer Ausführungsform ist eine (C₁-C₆)-Alkandiylgruppe, die in X vorkommt, eine (C₁-C₄)-Alkandiylgruppe, in einer anderen Ausführungsform eine (C₁-C₃)-Alkandiyl-gruppe, in einer anderen Ausführungsform eine (C₁-C₂)-Alkandiylgruppe. Beispiele für Gruppen X, aus denen die jeweilige X repräsentierende Gruppe in den oben aufgeführten Ausführungsformen gewählt sein kann oder aus denen in einer anderen Ausführungsform der Erfindung X ausgewählt sein kann, sind Methylen, -CH(CH₃)-(Ethan-1,1-diyl), -CH₂-CH₂-(Ethan-1,2-diyl, 1,2-Ethylen), -C(CH₃)₂-(1-Methylethan-1,1-diyl),] -CH₂-CH₂-CH₂-(Propan-1,3-diyl, 1,3-Propylen), -CH₂-CH(CH₃)- und -CH(CH₃)-CH₂- (Propan-1,2-diyl, 1,2-Propylen), die die Gruppe (C₁-C₆)-Alkandiyl exemplifizieren. So ist in einer Ausführungsform X ausgewählt aus -CH₂-O-, -CH₂-CH₂-O-, -CH(CH₃)-O- und -C(CH₃)₂-O-, in einer anderen Ausführungsform aus -CH₂-O-, -CH₂-CH₂-O- und -CH(CH₃)-O-, in einer anderen Ausführungsform aus -CH₂-O- und - CH(CH₃)-O-, und in einer anderen Ausführungsform steht X für -CH₂-O- und wobei das Sauerstoffatom an die Gruppe Y gebunden ist.

In einer Ausführungsform der Erfindung ist die Gruppe R¹ aus Wasserstoff und (C₁-C₄)-Alkyl ausgewählt, in einer anderen Ausführungsform ist R¹ aus Wasserstoff, Methyl, Ethyl, n-Propyl, n-Butyl und Isopropyl ausgewählt, in einer anderen Ausführungsform aus Wasserstoff, Methyl und Ethyl, in einer anderen Ausführungsform steht R¹ für Wasserstoff, in einer anderen Ausführungsform steht R¹ für (C₁-C₄)-Alkyl, in einer anderen Ausführungsform steht R¹ für Methyl.

In einer Ausführungsform der Erfindung ist Y aus Phenylen ausgewählt. In einer Ausführungsform steht Y für Phenylen, wobei das Phenylen gegebenenfalls an einem oder mehreren Ringatomen durch gleiche oder verschiedene Substituenten R⁵ substituiert ist.

Die Ringkohlenstoffatome, über die die Phenylengruppe die Y repräsentieren, an den Oxazolopyrimidinring und die Gruppe X gebunden sind, können in beliebigen Positionen vorliegen. Eine Y repräsentierende Phenylengruppe kann 1,2-Phenylen sein, d.h. der Oxazolopyrimidinring und die Gruppe X können in 1,2-Position oder ortho-Position zueinander gebunden sein, sie kann 1,3-Phenylen sein, d.h. der Oxazolopyrimidinring und die Gruppe X können in 1,3-Position oder meta-Position zueinander gebunden sein, und sie kann 1,4-Phenylen sein, d.h. der Oxazolopyrimidinring und die Gruppe X können in 1,4-Position oder para-Position zueinander gebunden sein. In einer Ausführungsform ist eine Y repräsentierende Phenylengruppe ausgewählt aus 1,3-Phenylen und 1,4-Phenylen, in einer anderen Ausführungsform ist sie 1,3-Phenylen, und in einer anderen Ausführungsform ist sie 1,4-Phenylen, wobei alle diese Gruppen gegebenenfalls wie in bezug auf Y angegeben substituiert sind. In einer Ausführungsform ist die Zahl der Substituenten R⁵, die gegebenenfalls an Ringkohlenstoffatomen in Y vorliegen können, 1, 2, 3, 4 oder 5, in einer anderen Ausführungsform 1, 2, 3 oder 4, in einer anderen Ausführungsform 1, 2 oder 3, in einer anderen Ausführungsform 1 oder 2, in einer anderen Ausführungsform 1. Ringkohlenstoffatome in Y, die keinen Substituenten R⁵ tragen, tragen ein Wasserstoffatom.

In einer Ausführungsform der Erfindung sind die Substituenten R⁵, die gegebenenfalls an der Gruppe Y vorliegen, aus (C₁-C₄)-Alkyl ausgewählt.

In einer Ausführungsform sind 1, 2 oder 3 der Substituenten R⁵, in einer anderen Ausführungsform 1 oder 2 der Substituenten R⁵ und in einer anderen Ausführungsform 1 der Substituenten R⁵, die gegebenenfalls an der Gruppe Y vorliegen, wie in der allgemeinen Definition von R⁵ definiert und somit aus (C₁-C₄)-Alkyl-, ausgewählt, wobei alle Alkylgrupppen unabhängig voneinander gegebenenfalls durch einen oder mehrere Fluorsubstituenten substituiert sind, wie es allgemein für Alkylgruppen gilt. In einer Ausführungsform sind die Substituenten R⁵, die gegebenenfalls an der Gruppe Y vorliegen und in der oben aufgeführten Ausführungsform wie in der allgemeinen Definition von R⁵ definiert sind, beispielsweise 1 oder 2 derartige Substituenten R⁵ oder 1 derartiger Substituent R⁵, aus (C₁-C₄)-Alkyl-, ausgewählt. In einer Ausführungsform befinden sich die Substituenten R⁵, die gegebenenfalls an der Gruppe Y vorliegen und in der oben aufgeführten Ausführungsform wie in der allgemeinen Definition von R⁵ definiert sind, beispielsweise 1 oder 2 derartige Substituenten R⁵ oder 1 derartiger Substituent R⁵, nicht an Ringkohlenstoffatomen in der Gruppe Y, die dem Atom, über das die Gruppe Y an den in Formel I dargestellten Oxazolopyrimidinring gebunden ist, benachbart ist.

Gegenstand der Erfindung sind alle Verbindungen der Formel I, worin ein oder mehrere Strukturelemente wie Gruppen, Substituenten und Zahlen wie in einer der angegebenen Ausführungsformen oder Definitionen der Elemente definiert sind oder eine oder mehrere der spezifischen Bedeutungen, die hier als Beispiele für Elemente angegeben sind, besitzen, wobei alle Kombinationen einer oder mehrerer angegebener Ausführungsformen und/oder Definitionen und/oder spezifischer Bedeutungen der Elemente Gegenstand der vorliegenden Erfindung sind. Auch in bezug auf alle derartigen Verbindungen der Formel I sind alle ihre stereoisomeren Formen und Mischungen von stereoisomeren Formen in beliebigem Verhältnis und ihre physiologisch annehmbaren Salze und die physiologisch annehmbaren Solvate davon Gegenstand der vorliegenden Erfindung.

Eine weitere Ausführungsform betrifft Verbindungen der Formel I, worin ein oder mehrere Reste die folgenden Bedeutungen haben:
X -(CH₂)-O-, wobei -O- an die Gruppe Y gebunden ist;
Y Phenylen, wobei das Phenylen gegebenenfalls an einem oder mehreren Ringkohlenstoffatomen durch gleiche oder verschiedene Substituenten R⁵ substituiert ist;
R¹ Wasserstoff;
R² und R³ unabhängig voneinander ausgewählt sind aus H, Halogen;
R⁵ (C₁-C₄)-Alkyl.

Ebenso gilt auch in bezug auf alle hier offenbarten spezifischen Verbindungen, wie die Beispielverbindungen, die Ausführungsformen der Erfindung repräsentieren, in denen die verschiedenen Gruppen und Zahlen in der allgemeinen Definition der Verbindungen der Formel I die in der jeweiligen spezifischen Verbindung vorliegenden spezifischen Bedeutungen besitzen, daß sie in einer beliebigen ihrer stereoisomeren Formen und/oder einer Mischung von stereoisomeren Formen in beliebigem Verhältnis und in Form ihrer physiologisch akzeptablen Salze und in Form der physiologisch akzeptablen Solvate derartiger Verbindungen oder derartiger Salze Gegenstand der vorliegenden Erfindung sind. Unabhängig davon, ob eine spezifische Verbindung hier als freie Verbindung und/oder als spezifisches Salz offenbart wird, ist sie sowohl in Form der freien Verbindung als auch in Form aller ihrer physiologisch akzeptablen Salze und bei Offenbarung eines spezifischen Salzes zusätzlich in Form dieses spezifischen Salzes und in Form der physiologisch akzeptablen Solvate einer derartigen Verbindung oder derartiger Salze Gegenstand der Erfindung. Gegenstand der Erfindung ist somit auch eine Verbindung der Formel I, die aus einer oder mehreren der hier offenbarten spezifischen Verbindungen der Formel I einschließlich der nachstehend angeführten Beispielverbindungen ausgewählt ist, und die physiologisch akzeptablen Salze davon und die physiologisch akzeptablen Solvate einer derartigen Verbindung oder derartiger Salze, wobei die Verbindung der Formel I in einer beliebigen ihrer stereoisomeren Formen oder als Mischung von stereoisomeren Formen in beliebigem Verhältnis, sofern anwendbar, Gegenstand der Erfindung ist. Als Beispiel genannt ist eine Verbindung der Formel I oder ein physiologisch akzeptables Solvat davon, z.B. {4-[6-(2-Fluoro-phenoxy)-oxazolo[4,5-c]pyridin-2-yl]-2,6-dimethyl-phenoxy}-essigsäure.

Ein weiterer Gegenstand der vorliegenden Erfindung sind Verfahren zur Herstellung der Verbindungen der Formel I und ihrer Salze und Solvate, nach denen die Verbindungen erhältlich sind und die im Folgenden umrissen werden.

Bei einem Verfahren setzt man eine Verbindung der Formel II mit einer Verbindung der Formel III zu einer Verbindung der Formel I um, 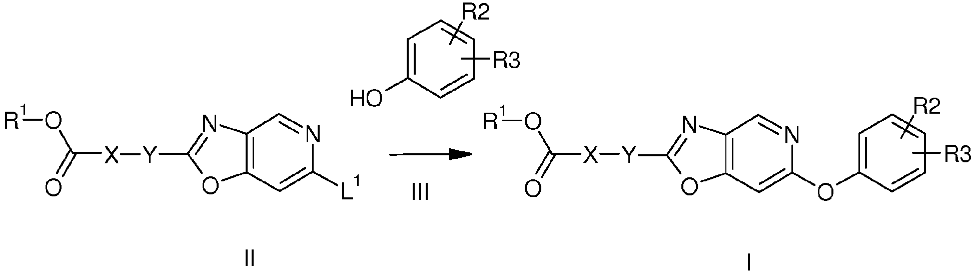 worin die Gruppen X, Y, R¹,R² und R³ in den Verbindungen der Formeln II und III wie in den Verbindungen der Formel I definiert sind und zusätzlich funktionelle Gruppen in geschützter Form oder in Form einer Vorläufergruppe, die später in die endgültige Gruppe umgewandelt wird, vorliegen können. Die Gruppe L¹ in den Verbindungen der Formel II ist eine Abgangsgruppe, die in einer, ggf. katalysierten, nukleophilen aromatischen Substitutionsreaktion ausgetauscht werden kann, wie ein Halogenatom, beispielsweise Fluor, Chlor oder Brom, oder eine Sulfongruppe, beispielsweise eine Gruppe der Formel -S(O)₂-Alk, worin Alk eine (C₁-C₄)-Alkylgruppe, beispielsweise Methyl oder Ethyl, ist.

Die Reaktion der Verbindungen der Formeln II und III ist eine, ggf. katalyiserte, nukleophile aromatische Substitutionsreaktion an dem Kohlenstoffatom in der 6-Position des Oxazolo[4,5-c]pyridinrings, d.h. in der Pyridingruppierung, und kann unter Standardbedingungen für derartige Reaktionen, die dem Fachmann gut bekannt sind, durchgeführt werden. Die Reaktion kann auch in Gegenwart von Katalysatorsystemen, z.B. Natriumtolylsulfinat oder Kupfer- oder Palladium-salzen oder -komplexen, durchgeführt werden. Im allgemeinen wird die Reaktion in einem inerten Lösungsmittel, beispielsweise einem Kohlenwasserstoff oder chlorierten Kohlenwasserstoff wie Benzol, Toluol, Xylol, Chlorbenzol, Dichlormethan, Chloroform oder Dichlorethan, einem Ether wie Tetrahydrofuran (THF), Dioxan, Dibutylether, Diisopropylether oder 1,2-Dimethoxyethan (DME), einem Keton wie Aceton oder Butan-2-on, einem Ester wie Essigsäureethylester oder Essigsäurebutylester, einem Nitril wie Acetonitril, einem Amin wie N,N-Dimethylformamid (DMF), N,N-Dimethylacetamid (DMA) oder N-Methylpyrrolidin-2-on (NMP) oder einer Lösungsmittelmischung bei Temperaturen von etwa 20°C bis etwa 250°C, beispielsweise bei Temperaturen von etwa 40°C bis etwa 200°C, je nach den Besonderheiten des jeweiligen Falls, durchgeführt. Im allgemeinen ist es günstig, zur Erhöhung der Reaktivität eine Base zuzusetzen, beispielsweise ein tertiäres Amin, wie Triethylamin, Ethyldiisopropylamin oder N-Methylmorpholin, oder eine anorganische Base wie ein Erdalkalimetallhydrid, -hydroxid, -carbonat oder - hydrogencarbonat wie Natriumhydrid, Natriumhydroxid, Kaliumhydroxid, Natriumcarbonat, Kaliumcarbonat, Cäsiumcarbonat oder Natriumhydrogencarbonat oder ein Alkoxid oder Amid wie Natriummethoxid, Natriumethoxid, Kaliummethoxid, Kalium-tert.-butoxid, Natriumamid oder Lithiumdiisopropylamid. Eine Verbindung der Formel III kann auch vor der Reaktion mit der Verbindung der Formel II separat mit einer Base behandelt und in ein Salz umgewandelt werden. Wenn die Reaktion in Gegenwart eines Katalysatorsystems durchgeführt wird, können hierbei Katalysatoren zu Einsatz kommen, die ein Metallion oder ein Metall in der Oxidationstufe 0 enthalten können, hierbei kommen bevorzugt Edelmetalle oder Edelmetallsalze zum Einsatz, hierunter wiederum bevorzugt sind Palladium und Kupfer. Die Katalyse erfordert häufig die Gegenwart bestimmter metall-komplexierender Liganden, die die Bildung einer katalytisch aktiven Spezies erst ermöglichen oder sie stabilisieren. Metall-Ligand-Komplexe können der Reaktion zugegeben werden oder in situ gebildet werden. Beispielsweise können solche Katalyssatorsysteme Kupfer oder Kupfer(i)salze, besonders Kupfer(I)halogenide oder Kupfer(I)carboxylate, insbesondere Kupfer(i)iodid oder Kupfer(I)thiophencarboxylat, oder auch vorgebildete Kupfer(I)-Komplexe, z.B. Tetrakis(aceotnitril)kupfer(I)hexafluorophoshat, allein oder in Gegenwart von Liganden, z.B. Diaminliganden oder 1,10-Phenanthrolin, enthalten. Des weiteren können solche Katalyssatorsysteme zum Beispiel aus Palladium-komplexen oder Palladiumsalzen in Gegenwart von Liganden, z.B. aus Palladium(0)komplexen, insbesondere Tris(dibenzylidenaceton)dipalladium(O), oder Palladiumacetat, Palladiumtrifluoracetat oder Palladiumhalogeniden, insbsondere Palladiumchlorid, in Gegenwart von Liganden, insbesondere Diphosphinliganden wie z.B. 2,2'-Bis(diphenylphosphino)-1-1'-binapthyl oder 4,5-Bis(diphenylphosphino)-9,9-dimethylxanthen oder vorgebildeten Komplexen wie Bis(tri-tert-butylphosphin)palladium(O) bestehen oder gebildet werden. Des weiteren können auch einfache Katalysatoren zum Einsatz kommen, z. B. kann die nucleophile aromatische Substitution von 2-Pyridinhalogeniden, insesondere -chloriden durch substituierte Alkali- oder Erdalkali-Benzolsulfinate, insbesondere durch Natriumtolylsulfinat, katalysiert werden.

Die Ausgangsverbindungen der Formeln II und III sind nach in der Literatur beschriebenen Verfahrensweisen oder in Anlehnung daran erhältlich und in vielen Fällen im Handel erhältlich. Die Verbindungen der Formel II sind beispielsweise durch Umsetzung eines 3-Amino-pyridinderivats der Formel IV mit einem aktivierten Carbonsäurederivat der Formel V zu einer Verbindung der Formel VI, Cyclisierung der letzteren Verbindung unter Bildung des Oxazolo[4,5-c]pyridinringsystems zu einer Verbindung der Formel VII, und Einführung der Gruppierung R¹O-C(O)-X- in die Verbindung der Formel VII durch Umsetzung mit einer Verbindung der Formel VIII zu einer Verbindung der Formel IX, die in Abhängigkeit der Bedeutung von R' und L¹ schon eine Verbindung der Formel II sein kann, und gegebenenfalls Modifizieren der Gruppe R' in der Verbindung der Formel IX unter Erhalt von Verbindungen der Formel II, erhältlich.

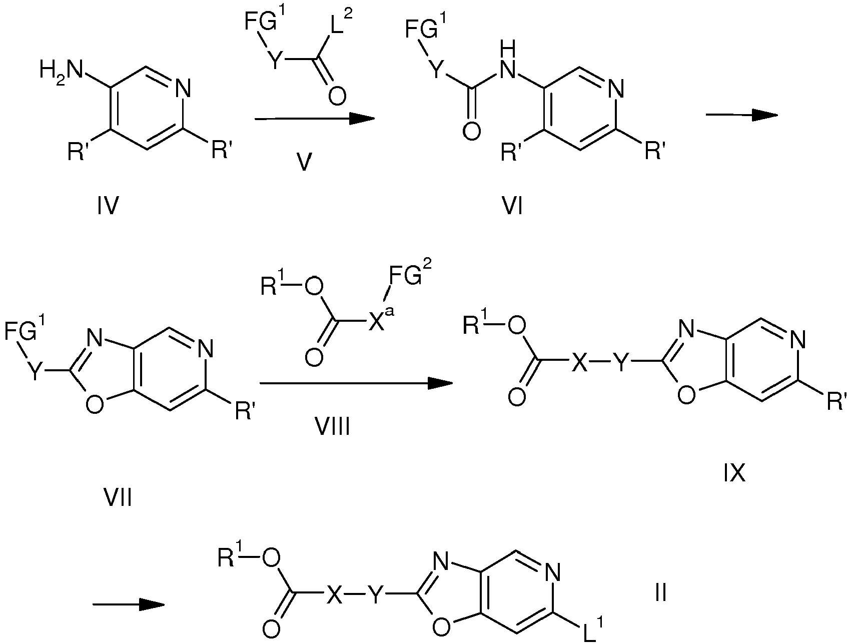

Die Gruppen X, Y und R¹ in den Verbindungen der Formeln II, V, VI, VII, VIII und IX sind wie in den Verbindungen der Formel I definiert, und zusätzlich können funktionelle Gruppen in geschützter Form oder in Form einer Vorläufergruppe, die später in die endgültige Gruppe umgewandelt wird, vorliegen. Die Gruppe X^{a} in den Verbindungen der Formel VIII ist wie die Gruppe X in den Verbindungen der Formel I definiert oder umfaßt einen Teil der Gruppe X in der gewünschten Verbindung der Formel II, so daß nach der Umsetzung der Verbindungen der Formeln VII und VIII die Gruppe X^{a} und jegliche in der Verbindung der Formel IX verbleibende Teile der Gruppen FG¹ und FG² zusammen die gewünschte Gruppe X bilden. So kann beispielsweise in dem Fall, daß die Gruppe X für eine Alkandiyloxygruppe steht, die Gruppe X^{a} in der Verbindung der Formel VIII die gewünschte Alkandiyloxygruppe sein und die Gruppe FG² kann ein an das Sauerstoffatom gebundenes Wasserstoffatom sein, oder die Gruppe X^{a} kann der Alkandiylteil sein, die Gruppe FG² ist eine Abgangsgruppe, und die Gruppe FG¹ in der Verbindung der Formel VII ist eine Hydroxygruppe, deren Sauerstoffatom zusammen mit dem Alkandiylteil dann nach Alkylierung der Verbindung der Formel VII mit der Verbindung der Formel VIII die gewünschte Alkandiyloxygruppe bildet.

Die Gruppen FG¹ und FG² in den Verbindungen der Formeln V, VI, VII und VIII sind funktionelle Gruppen, die für den zur Bildung der gewünschten Gruppe X aus der Gruppe X^{a} und jeglichem in der Verbindung der Formel IX verbleibenden Teil der Gruppen FG¹ und FG² verwendeten Kupplungstyp geeignet sind. Beispielsweise kann es sich dann, wenn die Gruppe X^{a} über eine nukleophile Substitutionsreaktion an die Gruppe Y oder an ein Atom in der Gruppe FG¹, wie ein Sauerstoffatom in einer Hydroxygruppe, die FG¹ repräsentiert, wie oben erwähnt, gebunden wird, bei FG² um eine Abgangsgruppe wie ein Halogenatom wie Chlor, Brom oder lod oder eine Sulfonyloxygruppe wie Methansulfonyloxy, Trifluormethansulfonyloxy oder Toluolsulfonyloxy handeln. Im Allgemeinen liegt die Gruppe FG¹ an dem Kohlenstoffatom in der Phenylengruppe oder heterocyclischen Gruppe, die Y repräsentiert, die in den Verbindungen der Formeln IX, II und I die Gruppe X trägt, vor. Die Gruppe FG¹ in den Verbindungen der Formeln V, VI und VII kann auch in geschützter Form oder in Form einer Vorläufergruppe, die später in die Gruppe umgewandelt wird, die in der Verbindung der Formel VII mit der Verbindung der Formel VIII reagiert, vorliegen. So kann beispielsweise eine Hydroxygruppe, die in der Verbindung der Formel VII FG¹ repräsentiert, in den Verbindungen der Formeln V und VI in geschützter Form vorliegen, beispielsweise in Form einer veretherten Hydroxygruppe wie eines Benzylethers oder eines Alkylethers wie eines Methylethers. Derartige Ether können nach dem Fachmann gutbekannten Methoden gespalten werden. Eine Zusammenfassung von Methoden zur Abspaltung von Schutzgruppen findet sich in der Literatur, beispielsweise in P. J. Kocienski, Protecting Groups (Thieme Verlag, 1994), oder T. W. Greene und P. G. M. Wuts, Protective Groups in Organic Synthesis (John Wiley & Sons, 1999).

Die Gruppe L¹ in der Verbindung II ist wie oben beschrieben definiert.

Die Gruppe L² in den Verbindungen der Formel V ist eine nukleophil substituierbare Abgangsgruppe und kann insbesondere ein Halogenatom, wie Chlor oder Brom, sein, und die Verbindung der Formel V kann somit ein Carbonsäurehalogenid sein. L² kann auch eine Gruppe der Formel FG¹-Y-C(O)-O sein, und die Verbindung der Formel V kann somit beispielsweise ein Carbonsäureanhydrid sein.

Die Gruppe R' in den Verbindungen der Formeln IV, VI, VII und IX kann eine Hydroxygruppe oder ein Halogenatom, wie Chlor oder Brom, sein.

Verbindungen, die bei der Synthese der Verbindungen der Formel I vorkommen, wie die Verbindung der Formel IV, können auch in einer anderen tautomeren Form vorliegen, beispielsweise in der Ketoform, wenn die Gruppen R' in der Verbindung der Formel IV Hydroxygruppen sind. Verbindungen, die bei der Synthese der Verbindungen der Formel I vorkommen, einschließlich Ausgangsverbindungen, Zwischenprodukten und Produkten, können auch in Form eines Salzes eingesetzt bzw. erhalten werden.

Die Umsetzung der Verbindungen der Formeln IV und V kann unter Standardbedingungen für die Acylierung eines Amins mit einem aktivierten Carbonsäurederivat wie einem Säurehalogenid oder -anhydrid durchgeführt werden. Im allgemeinen wird die Umsetzung in einem inerten Lösungsmittel, beispielsweise einem Kohlenwasserstoff oder chlorierten Kohlenwasserstoff wie Benzol, Toluol, Xylol, Chlorbenzol, Dichlormethan, Chloroform oder Dichlorethan, einem Ether wie THF, Dioxan, Dibutylether, Diisopropylether oder DME, einem Keton wie Aceton oder Butan-2-on, einem Ester wie Essigsäureethylester oder Essigsäurebutylester oder Wasser, oder einer Mischung von Lösungsmitteln, bei Temperaturen von etwa -10°C bis etwa 40°C, beispielsweise bei Temperaturen von etwa 0°C bis etwa 30°C durch-geführt. Im allgemeinen wird die Umsetzung unter Zugabe einer Base, beispielsweise eines tertiären Amins, wie Triethylamin, Ethyldiisopropylamin oder N-Methylmorpholin oder einer anorganischen Base wie eines Alkalimetall-hydroxids, -carbonats oder - hydrogencarbonats wie Natriumhydroxid, Kaliumhydroxid, Natriumcarbonat, Kaliumcarbonat oder Natriumhydrogencarbonat durchgeführt. Die Umsetzung der Verbindungen der Formeln VI und VII wird im allgemeinen in einem inerten Lösungsmittel, beispielsweise einem Alkohol, wie Methanol, Ethanol oder Isopropanol, oder einem Ether wie THF, Dioxan oder DME oder einer Mischung von Lösungsmitteln bei Temperaturen von etwa 20°C bis etwa 80°C, beispielsweise Temperaturen von etwa 40°C bis etwa 80°C, in Gegenwart einer Base, beispielsweise eines Alkoxids wie Natriummethoxid, Natriumethoxid, Kaliummethoxid oder Kalium-tert.-butoxid, durchgeführt.

Wenn die Gruppe R' in der Verbindung der Formel VI für Hydroxy steht, kann die Cyclisierung der Verbindung der Formel VI zu der Verbindung der Formel VII günstigerweise in Gegenwart eines Halogenierungsmittels wie eines Phosphorhalogenids, wie Phosphorpentachlorid oder Phosphoroxidchlorid oder einer Mischung davon, in einem inerten Lösungsmittel, beispielsweise einem Kohlenwasserstoff oder chlorierten Kohlenwasserstoff wie Benzol, Toluol, Xylol, Chlorbenzol, Dichlormethan, Chloroform oder Dichlorethan, bei Temperaturen von etwa 20°C bis etwa 100°C, beispielsweise Temperaturen von etwa 50°C bis etwa 80°C, durchgeführt werden. Wenn die Gruppe R' in der Verbindung der Formel VI für Halogen wie Chlor steht, kann die Cyclisierung der Verbindung der Formel VI zu der Verbindung der Formel VII thermisch durchgeführt werden, beispielsweise durch Erhitzen der Verbindung der Formel VI in einem inerten Lösungsmittel wie einem Kohlenwasserstoff oder chlorierten Kohlenwasserstoff, beispielsweise Toluol, Xylol oder Chlorbenzol oder einem Amid, beispielsweise DMF, DMA oder NMP, oder einem Nitril, beispielsweise Acetonitril, auf Temperaturen von etwa 100°C bis etwa 200°C, beispielsweise auf Temperaturen von etwa 120°C bis etwa 180°C, gegebenenfalls unter Druck und gegebenenfalls in Gegenwart einer Base, wie eines tertiären Amins, beispielsweise Triethylamin, Ethyldiisopropylamin oder N-Methylmorpholin, oder einer anorganischen Base, beispielsweise eines Alkalimetallhydroxids, -carbonats oder -hydrogencarbonats wie Natriumhydroxid, Kaliumhydroxid oder Natriumcarbonat, Kaliumcarbonat oder Natriumhydrogencarbonat. Die thermische Cyclisierung kann günstigerweise in einem Mikrowellenreaktor durchgeführt werden. Diese Cyclisierung kann auch in Gegenwart von Katalysatoren durchgeführt werden. Wenn die Reaktion in Gegenwart eines Katalysatorsystems durchgeführt wird, können hierbei Katalysatoren zu Einsatz kommen, die ein Metallion oder ein Metall in der Oxidationstufe 0 enthalten können, hierbei kommen bevorzugt Edelmetalle oder Edelmetallsalze zum Einsatz, hierunter wiederum bevorzugt sind Palladium und Kupfer. Die Katalyse erfordert häufig die Gegenwart bestimmter metall-komplexierender Liganden, die die Bildung einer katalytisch aktiven Spezies erst ermöglichen oder sie stabilisieren. Metall-Ligand-Komplexe können der Reaktion zugegeben werden oder in situ gebildet werden. Beispielsweise können solche Katalyssatorsysteme Kupfer oder Kupfer(I)salze, besonders Kupfer(I)halogenide oder Kupfer(I)carboxylate, insbesondere Kupfer(I)iodid oder Kupfer(I)thiophencarboxylat, oder auch vorgebildete Kupfer(I)-Komlplexe, z.B. Tetrakis(acetnitril)kupfer(I)hexafluorophoshat, allein oder in Gegenwart von Liganden, z.B. Diaminliganden oder 1,10-Phenanthrolin, enthalten. Des weiteren können solche Katalyssatorsysteme zum Beispiel aus Palladium-komplexen oder Palladiumsalzen in Gegenwart von Liganden, z.B. aus Palladium(0)komplexen, insbesondere Tris(dibenzylidenaceton)dipalladium(O), oder Palladiumacetat, Palladiumtrifluoracetat oder Palladiumhalogeniden, insbsondere Palladiumchlorid, in Gegenwart von Liganden, insbesondere Diphosphinliganden wie z.B. 2,2'-Bis(diphenylphosphino)-1-1'-binapthyl oder 4,5-Bis(diphenylphosphino)-9,9-dimethylxanthen oder vorgebildeten Komplexen wie Bis(tri-tert-butylphosphin)palladium(O) bestehen oder gebildet werden. Des weiteren können auch einfache Katalysatoren zum Einsatz kommen, z. B. kann die nucleophile aromatische Substitution von 2-Pyridinhalogeniden, insesondere -chloriden durch substituierte Alkali- oder Erdalkali-Benzolsulfinate, insbesondere durch Natriumtolylsulfinat, katalysiert werden.

Die Kupplung von Verbindungen der Formel VIII mit Verbindungen der Formel VII kann durch Reaktionen verschiedener Typen durchgeführt werden, wie oben bereits angegeben, beispielsweise über eine Alkylierungsreaktion. So kann die Gruppe Y beispielsweise dann, wenn sie eine Hydroxygruppe, die FG¹ repräsentiert, trägt, unter Verwendung einer Verbindung der Formel VIII, in der FG² für eine für nukleophile Substitutionsreaktionen geeignete Abgangsgruppe wie ein Halogenatom wie Chlor, Brom oder Iod oder eine Sulfonyloxygruppe wie Methansulfonyloxy oder Toluolsulfonyloxy steht, alkyliert werden. Die nukleophile Substitutionsreaktion an dem Kohlenstoffatom der Verbindung der Formel VIII, die die Gruppe FG² trägt, kann unter Standardbedingungen für derartige Reaktionen, die dem Fachmann gut bekannt sind, durchgeführt werden. Im Allgemeinen wird die Umsetzung je nach den Besonderheiten des jeweiligen Falls in einem inerten Lösungsmittel, beispielsweise einem Kohlenwasserstoff oder chlorierten Kohlenwasserstoff wie Benzol, Toluol, Xylol, Chlorbenzol, Dichlormethan, Chloroform oder Dichlorethan, einem Ether wie THF, Dioxan, Dibutylether, Diisopropylether oder DME, einem Alkohol wie Methanol, Ethanol oder Isopropanol, einem Keton wie Aceton oder Butan-2-on, einem Ester wie Essigsäureethylester oder Essigsäurebutylester, einem Nitril wie Acetonitril, einem Amid wie N,N-Dimethylformamid oder N-Methylpyrrolidin-2-on, oder einer Mischung von Lösungsmitteln bei Temperaturen von etwa 20°C bis etwa 100°C, beispielsweise bei Temperaturen von etwa 40°C bis etwa 80°C durchgeführt. Im Allgemeinen ist es günstig, zur Erhöhung der Nukleophilie der Verbindung der Formel XIII und/oder zur Bindung einer Säure, die bei der Umsetzung freigesetzt wird, eine Base, beispielsweise ein tertiäres Amin, wie Triethylamin, Ethyldiisopropylamin oder N-Methylmorpholin, oder eine anorganische Base wie Alkalimetallhydrid, -hydroxid, -carbonat oder - hydrogencarbonat wie Natriumhydrid, Natriumhydroxid, Kaliumhydroxid, Natriumcarbonat, Kaliumcarbonat, Cäsiumcarbonat oder Natriumhydrogencarbonat oder ein Alkoxid oder Amid wie Natriummethoxid, Natriumethoxid, Kaliummethoxid, Kalium-tert.-butoxid, Natriumamid oder Lithiumdiisopropylamid zuzusetzen. Eine Verbindung der Formel VII, worin FG¹ für Hydroxy steht, kann auch vor der Umsetzung mit der Verbindung der Formel VIII separat mit einer Base behandelt und in ein Salz umgewandelt werden. Eine Verbindung der Formel VII, worin FG¹ für Hydroxy steht, kann nicht nur durch Umsetzung mit einer Verbindung der Formel VIII, worin FG² für eine Abgangsgruppe wie angegeben steht, in eine Verbindung der Formel IX umgewandelt werden, sondern auch durch Umsetzung mit dem entsprechenden Alkohol, d.h. mit einer Verbindung der Formel VIII, worin FG² für Hydroxy steht, unter den Bedingungen der Mitsunobu-Reaktion in Gegenwart eines Azodicarboxylats wie Diethylazodicarboxylat oder Diisopropylazodicarboxylat und eines Phosphins wie Triphenylphosphin oder Tributylphosphin in einem inerten aprotischen Lösungsmittel, beispielsweise einem Ether wie THF oder Dioxan (s. O. Mitsunobu, Synthesis (1981), 1-28). Die Kupplung von Verbindungen der Formel VIII mit Verbindungen der Formel VII über eine übergangsmetallkatalysierte Reaktion kann auch unter den Bedingungen von palladiumkatalysierten Kreuzkupplungsreaktionen wie der Heck-, Stille- oder Suzuki-Kupplungsreaktion durchgeführt werden (siehe A. de Meijere und F. Diederich (Hrsg.), Metal-Catalyzed Cross-Coupling Reactions (Wiley-VCH, 2004)).

Die Verbindung der Formel IX kann bereits eine Verbindung der Formel II sein und bei der Umsetzung mit der Verbindung der Formel III eingesetzt werden, wenn sie aus einer Verbindung der Formel VI, worin R' für Halogen, wie Chlor, steht, erhalten worden ist und das Halogenatom im Cyclisierungsprodukt im Verlauf der Synthese nicht ersetzt worden ist, beispielsweise durch eine Hydroxygruppe während der Aufarbeitung, oder wenn sie aus einer Verbindung der Formel VI, worin R' für Hydroxy steht, erhalten worden ist und gleichzeitig mit der Cyclisierung die zweite Hydroxygruppe in der Verbindung der Formel VI bzw. VII halogeniert wird, also beispielsweise durch ein Chloratom ersetzt wird, wie es bei der Cyclisierung mit Hilfe eines Phosphorhalogenids oder Phosphoroxyhalogenids vorkommen kann. Wenn in der Verbindung der Formel IX R' für eine Hydroxygruppe steht, kann eine Verbindung der Formel IX unter Standardbedingungen in eine Verbindung der Formel II überführt werden, in der L¹ für ein Halogenatom, wie z.B. für ein Chlor, steht, beispielsweise durch Behandlung mit einem Halogenierungsmittel wie einem Phosphorhalogenid oder einem Phosphoroxyhalogenid. Je nach den Besonderheiten des speziellen Falls, wie der Reaktivität der spezifischen Verbindung der Formel III, die mit der Verbindung der Formel II umzusetzen ist, kann es auch vorteilhaft sein, die Gruppe R' in einer Verbindung der Formel IX zu modifizieren, selbst wenn es sich dabei bereits um eine Abgangsgruppe handelt. So kann man beispielsweise eine Verbindung der Formel IX, worin R' für Halogen, wie z.B. Chlor, steht, durch Behandlung mit einer Alkansulfinsäure der Formel Alk-S(O)-OH, worin Alk für (C₁-C₄)-Alkyl steht, in eine Verbindung der Formel II, worin L¹ für die Gruppe -S(O)₂-Alk steht, umwandeln. Eine derartige Umwandlung wird im allgemeinen in Gegenwart einer Base, wie eines Alkalimetallhydrids, -hydroxids, - carbonats oder -hydrogencarbonats wie Natriumhydrid, Natriumhydroxid, Kaliumhydroxid, Natriumcarbonat, Kaliumcarbonat, Cäsiumcarbonat oder Natriumhydrogencarbonat, in einem inerten Lösungsmittel, wie einem Kohlenwasserstoff wie Benzol, Toluol, Xylol oder Chlorbenzol, einem Ether wie THF, Dioxan, Dibutylether, Diisopropylether oder DME, einem Amid wie DMF oder NMP, oder einer Mischung von Lösungsmitteln bei Temperaturen von etwa 20°C bis etwa 250°C, beispielsweise bei Temperaturen von etwa 80°C bis etwa 200°C, durchgeführt. Eine Alkansulfinsäure kann auch vor der Umsetzung mit der Verbindung der Formel IX separat mit einer Base behandelt und in ein Salz umgewandelt werden.

Man kann auch die Reihenfolge der Schritte bei der Herstellung der Verbindungen der Formel I ändern und und beispielsweise eine Verbindung der Formel VIIa mit einer Verbindung der Formel III zu einer Verbindung der Formel X umsetzen, und das erhaltene Produkt X mit einer Verbindung der Formel VIII zu einer Verbindung der Formel I umsetzen, 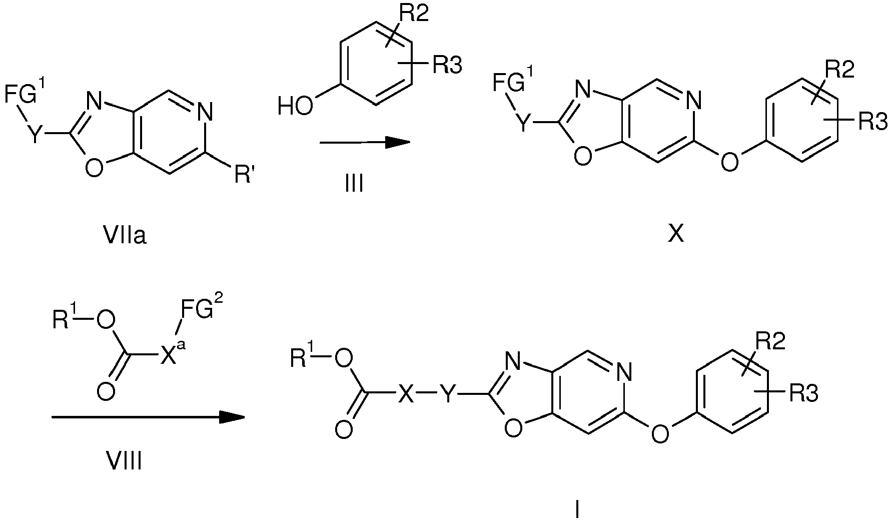 worin die Gruppen X, Y, R¹, R² und R³ in den Verbindungen der Formeln III, VIIa, VIII und X wie in den Verbindungen der Formel I definiert sind und zusätzlich funktionelle Gruppen in geschützter Form oder in Form einer Vorläufergruppe, die später in die endgültige Gruppe umgewandelt wird, vorliegen können, worin die Gruppe R' für ein Halogen, wie z.B. Chlor oder Brom steht, und worin die Gruppen X^{a}, FG¹ und FG² in den Verbindungen der Formeln VIIa, VIII und X wie oben definiert sind.

Die obigen Ausführungen zur Umsetzung der Verbindung der Formeln II und III und die Umsetzung der Verbindungen der Formeln VII und VIII gelten entsprechend für entsprechende Reaktionsschritte bei einer derartigen Synthese der Verbindungen der Formel I.

Weitere Verbindungen der Formel I sind aus geeigneten, nach den oben beschriebenen Verfahren hergestellten Verbindungen durch Funktionalisierung oder Modifizierung von enthaltenen funktionellen Gruppen nach Standardverfahrensweisen erhältlich, beispielsweise durch Veresterung, Amidierung, Hydrolyse, Veretherung, Alkylierung, Acylierung, Sulfonylierung, Reduktion, Oxidation, Umwandlung in Salze u.a. So kann beispielsweise eine Hydroxygruppe, die aus einer Ethergruppe durch Etherspaltung, beispielsweise mit Hilfe von Bortribromid, oder aus einer geschützten Hydroxygruppe durch Entschützung freigesetzt werden kann, zu einem Carbonsäureester oder einem Sulfonsäureester verestert oder verethert werden. Veretherungen von Hydroxygruppen können günstigerweise durch Alkylierung mit der jeweiligen Halogenverbindung, beispielsweise einem Bromid oder Iodid, in Gegenwart einer Base, beispielsweise eines Alkalimetallcarbonats wie Kaliumcarbonat oder Cäsiumcarbonat, in einem inerten Lösungsmittel, beispielsweise einem Amid wie DMF oder NMP oder einem Keton wie Aceton oder Butan-2-on oder mit dem jeweiligen Alkohol unter den oben angesprochenen Bedingungen der Mitsunobu-Reaktion durchgeführt werden. Eine Hydroxygruppe kann durch Behandlung mit einem Halogenierungsmittel in ein Halogenid umgewandelt werden. Ein Halogenatom kann in einer Substitutionsreaktion, bei der es sich auch um eine übergangsmetallkatalysierte Reaktion handeln kann, durch verschiedene Gruppen ersetzt werden. Eine Nitrogruppe kann zu einer Aminogruppe reduziert werden, beispielsweise durch katalytische Hydrierung. Eine Aminogruppe kann unter Standardbedingungen für die Alkylierung, beispielsweise durch Umsetzung mit einer Halogenverbindung oder durch reduktive Aminierung einer Carbonylverbindung, oder für die Acylierung oder Sulfonylierung, beispielsweise durch Umsetzung mit einem reaktiven Carbonsäurederivat wie einem Säurechlorid oder Anhydrid oder einem Sulfonsäurechlorid oder mit einer aktivierten Carbonsäure, die aus der Carbonsäure beispielsweise durch Behandlung mit einem Kupplungsmittel wie N,N'-Carbonyldiimidazol (CDI), einem Carbodiimid wie 1,3-Dicyclohexylcarbodiimid (DCC) oder 1-(3-Dimethylaminopropyl)-3-ethylcarbodiimid-hydrochlorid (EDC), O-(7-Azabenzotriazol-1-yl)-N,N,N',N'-tetramethyluroniumhexafluorophosphat (HATU), O-(Cyano(ethoxycarbonyl)methylenamino)-N,N,N',N'-tetramethyluroniumtetrafluoroborat (TOTU) oder [(Benzotriazol-1-yloxy)dimethylaminomethylen]di-methylammoniumtetrafluoroborat (TBTU) erhältlich ist, modifiziert werden. Eine Carbonsäureestergruppe kann unter sauren oder basischen Bedingungen zu einer Carbonsäure hydrolysiert werden. Eine Carbonsäuregruppe kann wie oben erwähnt aktiviert oder in ein reaktives Derivat umgewandelt und mit einem Alkohol oder einem Amin oder Ammoniak zu einem Ester oder Amid umgesetzt werden. Ein primäres Amid kann zu einem Nitril dehydratisiert werden. Ein Schwefelatom, beispielsweise in einer Alkyl-S-Gruppe oder in einem heterocyclischen Ring, kann mit einem Peroxid wie Wasserstoffperoxid oder einer Persäure zu einer Sulfoxidgruppierung S(O) oder einer Sulfongruppierung S(O)₂ oxidiert werden. Eine Carbonsäuregruppe, eine Carbonsäureestergruppe und eine Ketongruppe können zu einem Alkohol reduziert werden, beispielsweise mit Hilfe eines komplexen Hydrids wie Lithiumaluminiumhydrid, Lithiumborhydrid oder Natriumborhydrid. Eine Verbindung der Formel I oder ein Zwischenprodukt wie eine Verbindung der Formel II oder IX, die bzw. das eine Doppelbindung oder eine Dreifachbindung in der Gruppe X enthält, die über eine übergangsmetallkatalysierte Kupplungsreaktion leicht aus einer Verbindung der Formel VIII mit einer Doppel- oder Dreifachbindung in der Gruppe X^{a} und einer Verbindung der Formel VII wie oben beschrieben erhältlich ist, kann durch Hydrierung in Gegenwart von Hydrierkatalysator wie einem Palladiumkatalysator in eine Verbindung überführt werden, in der X für eine gesättigte Gruppe steht.

Alle bei den oben beschriebenen Synthesen der Verbindungen der Formel I verwendeten Reaktionen sind dem Fachmann an sich gut bekannt und können unter Standardbedingungen gemäß oder in Analogie zu in der Literatur, beispielsweise in Houben-Weyl, Methoden der Organischen Chemie (Methods of Organic Chemistry), Thieme-Verlag, Stuttgart, oder Organic Reactions, John Wiley & Sons, New York, beschriebenen Verfahrensweisen durchgeführt werden. Falls gewünscht, können die erhaltenen Verbindungen der Formel I sowie etwaige Zwischenverbindungen nach herkömmlichen Reinigungsverfahrensweisen gereinigt werden, beispielsweise durch Umkristallisieren oder Chromatographie. Wie bereits erwähnt, können alle bei den oben beschriebenen Synthesen eingesetzten Ausgangsverbindungen und Zwischenprodukte, die eine saure oder basische Gruppe enthalten, auch in Form von Salzen eingesetzt werden und alle Zwischenprodukte und entgültigen Zielverbindungen können auch in Form von Salzen erhalten werden. Wie ebenfalls oben erwähnt, kann es je nach den Umständen des jeweiligen Falls zur Vermeidung eines unerwünschten Verlaufs einer Reaktion oder von Nebenreaktionen im Lauf der Synthese einer Verbindung im allgemeinen erforderlich oder vorteilhaft sein, funktionelle Gruppen durch die Einführung von Schutzgruppen zeitweilig zu blockieren und sie in einer späteren Stufe der Synthese wieder zu entschützen oder funktionelle Gruppen in Form von Vorläufergruppen, die später in die gewünschten funktionellen Gruppen umgewandelt werden, einzuführen. Als Beispiele für Schutzgruppen seien Aminoschutzgruppen genannt, bei denen es sich um Acylgruppen oder Alkyloxycarbonylgruppen, beispielsweise eine tert.-Butyloxycarbonylgruppe (=Boc), die durch Behandlung mit Trifluoressigsäure (=TFA) abgespalten werden kann, eine Benzyloxycarbonylgruppe, die durch katalytische Hydrierung abgespalten werden kann, oder eine Fluoren-9-ylmethoxycarbonylgruppe,die durch Behandlung mit Piperidin abgespalten werden kann, handeln kann, und Schutzgruppen von Carbonsäuregruppen, die als Estergruppen, wie tert.-Butylester, die durch Behandlung mit Trifluoressigsäure entschützt werden können, oder Benzylester, die durch katalytische Hydrierung entschützt werden können, geschützt werden können. Als Beispiel für eine Vorläufergruppe sei die Nitrogruppe genannt, die durch Reduktion, beispielsweise durch katalytische Hydrierung, in eine Aminogruppe umgewandelt werden kann. Derartige Synthesestrategien und Schutzgruppen und Vorläufergruppen, die in einem bestimmten Fall geeignet sind, sind dem Fachmann bekannt.

Ein weiterer Gegenstand der vorliegenden Erfindung sind die neuen Ausgangsverbindungen und Zwischenprodukte, die bei der Synthese der Verbindungen der Formel I vorkommen, einschließlich der Verbindungen der Formeln II, III, IV, V, VI, VII, VIII, IX und X, worin X, X^{a}, Y, R¹, R², R³, R', FG¹, FG², L¹ und L² wie oben definiert sind, in einer beliebigen ihrer stereoisomeren Formen oder einer Mischung von stereoisomeren Formen in beliebigem Verhältnis und ihre Salze und Solvate derartiger Verbindungen oder derartiger Salze und ihre Verwendung als Zwischenprodukte. Die Erfindung schließt auch alle tautomeren Formen der Zwischenprodukte und Ausgangsverbindungen ein. Alle oben bezüglich der Verbindungen der Formel I angegebenen Erklärungen und Ausführungsformen gelten entsprechend auch für die Zwischenprodukte und Ausgangsverbindungen. Gegenstand der Erfindung sind insbesondere die hier offenbarten neuen spezifischen Ausgangsverbindungen und Zwischenprodukte. Unabhängig davon, ob sie als freie Verbindung und/oder als spezifisches Salz offenbart sind, sind sie sowohl in Form der freien Verbindungen als auch in Form ihrer Salze und im Fall der Offenbarung eines spezifischen Salzes zusätzlich in Form dieses spezifischen Salzes und in Form von Solvaten derartiger Verbindungen oder derartiger Salze Gegenstand der Erfindung.

Die Verbindungen der Formel I, gegebenenfalls in Kombination mit anderen pharmakologisch wirksamen Verbindungen, können Tieren, vorzugsweise Säugetieren einschließlich Menschen, als Pharmazeutika für sich alleine, in Mischungen miteinander oder in Form pharmazeutischer Zusammensetzungen verabreicht werden. Die Verabreichung kann oral, beispielsweise in Form von Tabletten, Filmtabletten, Dragees, Granulaten, Hart- und Weichgelatinekapseln, Lösungen einschließlich wäßriger, alkoholischer und öliger Lösungen, Säften, Tropfen, Sirupen, Emulsionen oder Suspensionen, rektal, beispielsweise in Form von Suppositorien, oder parenteral, beispielsweise in Form von Lösungen zur subkutanen, intramuskulären oder intravenösen Injektion oder Infusion, insbesondere wäßrigen Lösungen, durchgeführt werden. Die Verbindungen der Formel I können des weiteren in Modi der lokalen Arzneistoffzufuhr verwendet werden, beispielsweise in beschichteten Stents zur Verhinderung oder Verringerung der In-Stent-Restenose oder durch lokale Anwendung mit Hilfe eines Katheters. Die geeignete Verabreichungsform hängt u.a. von der zu behandelnden Erkrankung und ihrer Schwere ab.

Die Verabreichung der Verbindungen der Formel I, kann auch topisch erfolgen. Geeignete pharmazeutische Zusammensetzungen für die topische Anwendung auf der Haut liegen als Salbe, Creme, Lotion, Paste, Gel, Hydrogel, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im Allgemeinen in einer Konzentration von 0,0001 bis 15 Gew.-% der Zusammensetzung vorhanden, beispielsweise von 0,0005 bis 2%.
In einer Ausführungsform liegt die topische Zubereitung als Gel vor.
In einer weiteren Ausführungsform liegt die topische Formulierung als Hydrogel vor.

Unter einem Hydrogel wird ein Wasser enthaltendes, aber wasserunlösliches Polymer, dessen Moleküle chemisch, z. B. durch kovalente oder ionische Bindungen, oder physikalisch, z. B. durch Verschlaufen der Polymerketten, zu einem dreidimensionalen Netzwerk verknüpft sind verstanden. Durch eingebaute hydrophile Polymerkomponenten quellen sie in Wasser unter beträchtlicher Volumenzunahme, ohne aber ihren stofflichen Zusammenhalt zu verlieren. Ein Hydrogel besteht beispielsweise aus einem hydrophilen Lösungsmittel (z.B. Wasser), einem Feuchthaltemittel (z.B. Glycerol) und einem Gelbildner (z.B. Croscarmellose-Natrium).

Die folgenden Beispiele zeigen geeignete Gelzubereitungen:

**Zubereitungsbeispiel 1**

| | |
|---|---|
| Verbindung aus Beispiel 1 | 0.0004% |
| Glycerol 85% | 10% |
| Methylparaben | 0.2% |
| Propylparaben | 0.03% |
| Croscarmellose-Natrium | 4% |
| HCl / NaOH | qs (zum Einstellen auf pH 7.5) |
| Wasser | ad 100% |

**Zubereitungsbeispiel 2**

| | |
|---|---|
| Verbindung aus Beispiel 1 | 0.04% |
| Glycerol 85% | 10% |
| Methylparaben | 0.2% |
| Propylparaben | 0.03% |
| Croscarmellose-Natrium | 4% |
| HCl / NaOH | qs (zum Einstellen auf pH 7.5) |
| Wasser | ad 100% |

**Zubereitungsbeispiel 3**

| | |
|---|---|
| Verbindung aus Beispiel 1 | 0.0004% |
| PEG400 | 10% |
| Methylparaben | 0.2% |
| Propylparaben | 0.03% |
| Croscarmellose-Natrium | 4% |
| HCl / NaOH | qs (zum Einstellen auf pH 7.5) |
| Wasser | ad 100% |

**Zubereitungsbeispiel 4**

| | |
|---|---|
| Verbindung aus Beispiel 1 | 0.04% |
| PEG400 | 10% |
| Methylparaben | 0.2% |
| Propylparaben | 0.03% |
| Croscarmellose-Natrium | 4% |
| HCl / NaOH | qs (zum Einstellen auf pH 7.5) |
| Wasser | ad 100% |

Die Hydrogele sind Zubereitung zur dermalen Anwendung. Die Hydrogele können auf offene Wundareale aufgetragen werden. Die Hydrogele enthalten den Arzneistoff in gelöster Form, wodurch eine schnelle Haut- und Gewebepentration gewährleistet ist. Durch einen aseptischen Herstellprozess wird gewährleistet, dass durch die Applikation des Arzneimittels keine zusätzlichen mikrobiologischen Verunreinigungen in die Wunde gelangen. In einer Ausführungsform werden zusätzlich in das Hydrogel Konservierungsmittel (Methyl- und Propylparabene) eingearbeitet, um die Keimbelastung gering zu halten.

In einer Ausführungsform enthält das Hydrogel die Verbindungen der Formel I, in Konzentrationen von 0.04 - 0.0004% (m/m).

Das aseptische Hydrogel wird in geigneten sterilen Behältern gelagert. In einer Ausführungsform wird das Hydrogel in sterilen Behältern aus Polypropylen gelagert.

Die Menge einer Verbindung der Formel I und/oder ihrer physiologisch akzeptablen Salze und/oder Solvate in den pharmazeutischen Zusammensetzungen liegt normalerweise im Bereich von etwa 0,2 bis etwa 800 mg, beispielsweise von etwa 0,5 bis etwa 500 mg, beispielsweise von etwa 1 bis etwa 200 mg, pro Einheitsdosis, kann aber je nach Art der pharmazeutischen Zusammensetzung auch höher sein. Die pharmazeutischen Zusammensetzungen enthalten in der Regel etwa 0,5 bis etwa 90 Gew.-% der Verbindung der Formel I und/oder ihrer physiologisch akzeptablen Salze und/oder Solvate. Die pharmazeutischen Zusammensetzungen können auf an sich bekannte Art und Weise hergestellt werden. Hierzu bringt man eine oder mehrere Verbindungen der Formel I und/oder ihre physiologisch akzeptablen Salze und/oder Solvate zusammen mit einer oder mehreren festen oder flüssigen pharmazeutischen Trägersubstanzen oder Vehikeln und/oder Additiven oder Hilfssubstanzen und dann, wenn ein Kombinationsmedikament gewünscht ist, anderen pharmakologisch wirksamen Verbindungen mit therapeutischer oder prophylaktischer Wirkung in eine für die Verabreichung und Dosierung geeignete Form, die dann in der Human- oder Tiermedizin verwendet werden kann. Als Trägersubstanzen und Additive können geeignete organische und anorganische Substanzen verwendet werden, die mit den Verbindungen der Formel I oder ihren physiologisch akzeptablen Salzen oder Solvaten nicht in unerwünschter Weise reagieren. Als Beispiele für Additivtypen, die in den pharmazeutischen Zusammensetzungen und Medikamenten enthalten sein können, seien Gleitmittel, Konservierungsstoffe, Verdicker, Stabilisatoren, Sprengmittel, Netzmittel, Mittel zur Erzielung eines Depoteffekts, Emulgatoren, Salze, beispielsweise zur Beeinflussung des osmotischen Drucks, Puffersubstanzen, Farbmittel, Geschmacksstoffe und aromatische Substanzen genannt. Beispiele für Trägersubstanzen und Additive sind Wasser, physiologische Natriumchloridlösung, Pflanzenöle, Wachse, Alkohole wie Ethanol, Isopropanol, 1,2-Propandiol, Benzylalkohole oder Glycerin, Polyole, Mannit, Polyethylenglykole, Polypropylenglykole, Glycerintriacetat, Polyvinylpyrrolidon, Gelatine, Cellulose, Kohlenhydrate wie Lactose, Glucose, Saccharose oder Stärke wie Maisstärke, Stearinsäure und Stearinsäuresalze wie Magnesiumstearat, Talk, Lanolin, Vaseline oder Mischungen davon, beispielsweise Mischungen von Wasser mit einem oder mehreren organischen Lösungsmitteln wie Mischungen von Wasser mit Alkoholen. Man kann die Verbindungen der Formel I und ihre physiologisch akzeptablen Salze und Solvate auch lyophilisieren und die erhaltenen Lyophilisate beispielsweise zur Herstellung von Injektionszusammensetzungen verwenden.
Die Dosierung einer zu verabreichenden Verbindung der Formel I und/oder eines physiologisch akzeptablen Salzes und/oder Solvats davon hängt vom Einzelfall ab und ist wie üblich zur Erzielung einer optimalen Wirkung vom Arzt nach den üblichen Regeln und Verfahrensweisen den individuellen Gegebenheiten anzupassen. So hängt sie beispielsweise ab von der Art und Schwere der zu behandelnden Störung, von Geschlecht, Alter, Gewicht und individueller Ansprechbarkeit des zu behandelnden Menschen oder Tiers, von der Effizienz und Wirkdauer der verwendeten Verbindung, davon, ob die Behandlung für die Therapie einer akuten oder chronischen Erkrankung oder prophylaktisch ist, oder davon, ob neben einer Verbindung der Formel I weitere Wirkstoffe verabreicht werden. Im allgemeinen ist eine Tagesdosis von beispielsweise etwa 0,01 mg/kg bis etwa 100 mg/kg oder von etwa 0,1 mg/kg bis etwa 10 mg/kg oder von etwa 0,3 mg/kg bis etwa 5 mg/kg (jeweils mg pro kg Körpergewicht) zur Verabreichung an einen 75 kg schweren Erwachsenen zur Erzielung der gewünschten Ergebnisse angemessen. Die Tagesdosis kann dabei als Einzeldosis verabreicht oder, insbesondere bei Verabreichung größerer Mengen, in mehrere, beispielsweise zwei, drei oder vier, Einzeldosen aufgeteilt werden. Die Verabreichung kann auch kontinuierlich durchgeführt werden, beispielsweise durch kontinuierliche Infusion oder Injektion. Im Einzelfall kann es je nach dem individuellen Verhalten erforderlich sein, von den angegebenen Dosierungen nach oben oder nach unten abzuweichen.

Die folgenden Beispiele erläutern die Erfindung.

Wenn Beispielsverbindungen mit einer basischen Gruppe durch präparative HochdruckFlüssigkeitschromatographie (HPLC) an Umkehrphasen-Säulenmaterial (RP-Säulenmaterial) gereinigt wurden und es sich bei dem Elutionsmittel wie üblich um eine Gradientenmischung von Wasser und Acetonitril mit Trifluoressigsäure (TFA) handelte, wurden sie je nach den Einzelheiten der Aufarbeitung wie Verdampfungs- oder Lyophilisierungsbedingungen zum Teil in Form ihres Säureadditionssalzes mit Trifluoressigsäure erhalten. In den Namen der Beispielverbindungen und ihren Strukturformeln ist jegliche derartige enthaltene Trifluoressigsäure nicht angegeben.

Die hergestellten Verbindungen wurden im allgemeinen durch spektroskopische Daten und chromatografische Daten, insbesondere Massenspektrum (MS) und HPLC-Retentionszeiten (Rt; in min), die durch kombinierte analytische HPLC/MS-Charakterisierung (LC/MS) erhalten wurden, und/oder NMR-Spektren (NMR=kernmagnetische Resonanz), charakterisiert. Bei der NMR-Charakterisierung sind die chemische Verschiebung δ (in ppm), die Zahl der Wasserstoffatome und die Multiplizität (s = Singulett, d = Dublett, dd = doppeltes Dublett, t = Triplett, dt = doppeltes Triplett, q = Quartett, m = Multiplett; br = breit) der Signale angegeben. Bei der MS-Charakterisierung ist im allgemeinen die Massenzahl (m/z) des Peaks des Molekülions M, z.B. M⁺, oder eines verwandten Ions wie des Ions M+1, z.B. [M+1]⁺, d.h. des protonierten Molekülions [M+H]⁺, das je nach der verwendeten Ionisierungsmethode gebildet wurde, angegeben. Bei der Ionisierungsmethode handelte es sich im Allgemeinen um Elektrospray-Ionisierung (ESI). Es wurden die folgenden LC/MS-Bedingungen verwendet.

### Methode LC1

Säule: Phenomenex, 4 µM, 10 x 2 mm, 1,7 µm; Durchfluß: 1,1 ml/min; Elutionsmittel A: Wasser + 0,05% Trifluoressigsäure; Elutionsmittel B: Acetonitril; Gradient: von 93% A + 7% B bis 5% A + 95% B in 1,2 min, dann 5% A + 95% B für 0,2 min; MSlonisationsmethode: ESI⁺

### Methode LC2

Säule: UPLC BEH C18, 50 x 2,1 mm, 1,7 µm; Durchfluß: 0,9 ml/min; Elutionsmittel A: Wasser + 0,1 % Ameisensäure; Elutionsmittel B: Acetonitril + 0,08% Ameisensäure; Gradient: von 95% A + 5% B bis 5% A + 95% B in 1,1 min, dann 5% A + 95% B für 0,6 min; MS-Ionisationsmethode: ESI⁺

### Methode LC3

Säule: UPLC BEH C18, 50 x 2,1 mm, 1,7 µm; Durchfluß: 0,9 ml/min; Elutionsmittel A: Wasser + 0,05% Ameisensäure; Elutionsmittel B: Acetonitril + 0,035 % Ameisensäure; Gradient: von 95% A + 5% B bis 5% A + 95% B in 1,1 min, dann 5% A + 95% B für 0,6 min; MS-Ionisationsmethode: ESI⁺

### Beispiel 1

### {4-[6-(2-Fluoro-phenoxy)-oxazolo[4,5-c]pyridin-2-yl]-2,6-dimethyl-phenoxy}-essigsäure

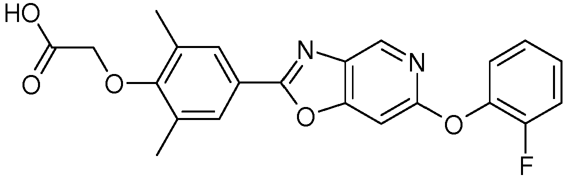

### (a) N-(4,6-Dichloro-pyridin-3-yl)-4-methoxy-3,5-dimethyl-benzamid

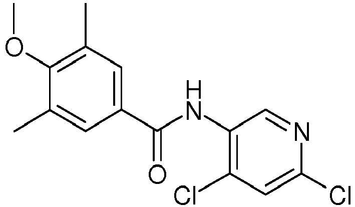

Zu einer Lösung von 0,82 g 4,6-Dichlor-pyridin-3-yl-amin in 8 ml trockenem Dichlormethan wurde unter Eiskühlung zunächst 1,20 g 4-Methoxy-3,5-dimethyl-benzoyl-chlorid, gelöst in 1 ml trockenem Dichlormethan zugetropft. Anschließend wurde eine Lösung von 0,4 ml absolutiertem Pyridin in 1 ml trockenem Dichlormethan zugegeben und die Reaktion für 2 h bei 0 °C gerührt, wobei das Produkt z.T. langsam ausfiel. Zur dieser Mischung wurden dann ca. 10 ml 10 %ige wässrige Natriumhydrogensulfatlösung gegeben und fünf Minuten lang gerührt. Anschließend wurde die wässrige Phase abdekantiert und die Feststoff enthaltende organische Phase im Vakuum eingeengt und gefriergetrocknet. Man erhielt 1,63 g (100 %) des Produktes, das ohne weitere Aufreinigung im nächsten Schritt eingesetzt wurde.
LC/MS (Methode LC1): Rt = 0,92 min; m/z = 325,05 [M+H]⁺

### (b) 6-Chlor-2-(4-methoxy-3,5-dimethyl-phenyl)-oxazolo[4,5-c]pyridin

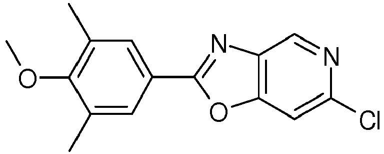

In einem Mikrowellengefäß wurden 0,98 g N-(4,6-Dichloro-pyridin-3-yl)-4-methoxy-3,5-dimethyl-benzamid in 15 ml trockenem Tetrahydrofuran gelöst und mit 29 mg Kupfer(I)iodid, 54 mg 1,10-Phenanthrolin und 1,47 g Cäsiumcarbonat versetzt. Das Reaktionsgemisch wurde dann in einem Mikrowellensynthesizer für 3 h auf 180 °C erhitzt. Zur Aufarbeitung wurde die Mischung auf 12 ml einer 0,5 M wässrigen Salzsäurelösung gegeben. Der ausgefallene Feststoff wurde abgesaugt und die Mutterlauge wurde zweimal mit Ethylacetat extrahiert. Die gesammelten organischen Phasen wurden mit dem abgesaugten Feststoff vereinigt und das Lösungsmittel wurde im Vakuum entfernt. Man erhielt 0,79 g (91 %)des Produktes, das ohne weitere Aufreinigung weiter umgesetzt wurde.
LC/MS (Methode LC1): Rt = 1,04 min; m/z = 289,05 [M+H]⁺

### (c) 6-(2-Fluoro-phenoxy)-2-(4-methoxy-3,5-dimethyl-phenyl)-oxazolo[4,5-c]pyridin

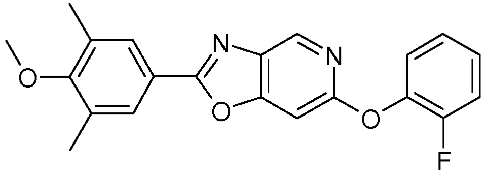

In einem Mikrowellengefäß wurden 0,65 g 6-Chlor-2-(4-methoxy-3,5-dimethyl-phenyl)-oxazolo[4,5-c]pyridin in 12 ml absolutiertem N,N-Dimethylformamid gelöst und mit 0,25 g 2-Fluorphenol, 0,88 g Cäsiumcarbonat, 21 mg Kupfer(I)iodid und 40 mg 1,10-Phenanthrolin versetzt. Die Reaktion wurde in einem Mikrowellenreaktor für 1 h auf 200 °C erhitzt. Zur Aufarbeitung wurde die Mischung auf gesättigte wässrige Nartiumhydrogencarbonatlösung gegeben und dreimal mit Ethylacetat extrahiert. Die vereinigten organischen Phasen wurden mit gesättigter wässriger Natriumhydrogensulfatlösung gewaschen, über Natriumsulphat getrocknet, filtriert und eingeengt. Das Rohprodukt wurde mittels präparativer HPLC gereinigt und man erhielt 86 mg (10%) des Produkts.
LC/MS (Methode LC2): Rt = 1,46 min; m/z = 365,13 [M+H]⁺

### (d) 4-[6-(2-Fluoro-phenoxy)-oxazolo[4,5-c]pyridin-2-yl]-2,6-dimethyl-phenol

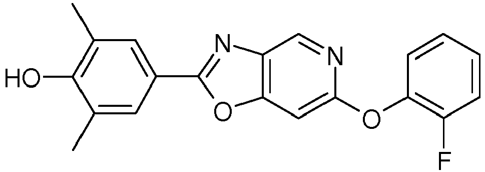

Eine Lösung von 60 mg 6-(2-Fluoro-phenoxy)-2-(4-methoxy-3,5-dimethyl-phenyl)-oxazolo[4,5-c]pyridin in 2 ml Dichlormethan wurde auf 0°C abgekühlt und über einen Zeitraum von 10 min mit 0,4 ml einer 1 M Lösung von Bortribromid in Dichlormethan versetzt. Die Mischung wurde 1 h bei 0°C gerührt und dann mit weiteren 0,2 ml einer 1 M Lösung von Bortribromid in Dichlormethan versetzt. Die Reaktion rührte dann 16 h bei Raumtemperatur. Zur Aufarbeitung wurde die Reaktionsmischung lansam mit gesättigter wäßriger Natriumhydrogencarbonatlösung versetzt. Der Niederschlag wurde abfiltriert, mit Wasser gewaschen und im Vakuum getrocknet. Man erhielt 41 mg (71 %) der Titelverbindung.
LC/MS (Methode LC2): Rt = 1,35 min; m/z = 351,13 [M+H]⁺

### (e) {4-[6-(2-Fluoro-phenoxy)-oxazolo[4,5-c]pyridin-2-yl]-2,6-dimethyl-phenoxy}-essigsäure tert-butyl ester

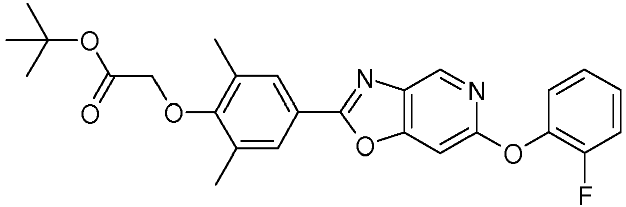

Eine Lösung von 70 mg 4-[6-(2-Fluoro-phenoxy)-oxazolo[4,5-c]pyridin-2-yl]-2,6-dimethyl-phenol in 1 ml Dimethylformamid wurde mit 110 mg Kaliumcarbonat versetzt, und dann wurden 51 mg Bromessigsäure-tert.-butylester zugegeben. Die Mischung wurde 16 h bei Raumtemperatur gerührt. Dann wurde Reaktionsansatz auf Wasser gegeben und zweimal mit Ethylacetat extrahiert. Die gesammelten organischen Phasen wurden getrocknet und eingeengt. Man erhielt 93 mg (100 %) der Titelverbindung, die ohne wietere Aufreinigung weiter umgesetzt wurde.
LC/MS (Methode LC2): Rt = 1,48 min; m/z = 465,20 [M+H]⁺

### (f) {4-[6-(2-Fluoro-phenoxy)-oxazolo[4,5-c]pyridin-2-yl]-2,6-dimethyl-phenoxy}-essigsäure

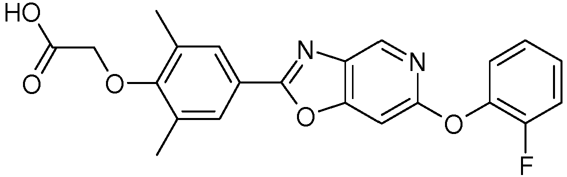

Eine Lösung von 90 mg {4-[6-(2-Fluoro-phenoxy)-oxazolo[4,5-c]pyridin-2-yl]-2,6-dimethyl-phenoxy}-essigsäure tert-butyl ester in 2 ml Dichlormethan wurde mit 0,8 ml Trifluoressigsäure versetzt und 2 h bei Raumtemperatur gerührt. Dann wurde der Ansatz im Vakuum eingeengt und gefriergetrocknet. Man erhielt 101 mg (100%) der Titelverbindung.
LC/MS (Methode LC3): Rt = 1,17 min; m/z = 409,18 [M+H]⁺

### Bestimmung der pharmakologischen Wirkung

### A) GTP-γ-S-Assay mit humanen Edg-1-Rezeptoren

Zur Bestimmung der Edg-1-Rezeptor-Aktivierung durch die erfindungsgemäßen Verbindungen wurde ein GTP-γ-S-Assay (GTP-γ-S = Guanosin-5'-[thio]triphosphat) auf die Bindung an G-Protein gekoppeltem Rezeptor auf Basis des Szintillationsproximitätsassay-Prinzips verwendet, wobei ein Zellmembranpräparat einer CHO-Flp-In-Zelllinie, die den humanen Edg-1-Rezeptor konstitutiv überexprimiert, eingesetzt wurde.

### (a) Erzeugung der Zelllinie

Das Flp-In™-Expressionssystem (Invitrogen, Kat.-Nr. K6010-01) erlaubt die Erzeugung von stabilen Säugetierzelllinien, in die das interessierende Gen durch homologe Rekombination an einem spezifischen Genomort, der als FRT-Ort (FRT = Flp Recombination Target) bezeichnet wird, mit Hilfe einer durch das pOG44-Expressionsplasmid codierten Flp-Rekombinase integriert worden ist. Die Integration des pcDNA5/FRT-Expressionskonstrukts in das Flp-In-Wirtszellliniengenom führt zur Transkription des interessierenden Gens. Die stabil transfizierten Zellen werden hygromycinresistent.

Einen Tag vor der Transfektion wurden 200 000 Flp-In-CHO-Zellen in Ham-F-12-Medium (Invitrogen, Kat.-Nr. 31765) mit 10% fötalem Kälberserum (FCS; Perbio Science, Kat.-Nr. SH30068.03) in einer Platte mit 6 Vertiefungen ausgesät und über Nacht bei 37°C/5 % CO₂ inkubiert. Unter Verwendung von FuGENE^{®}-6-Transfektionsreagens (Roche, Kat.-Nr. 11988387001) wurden Zellen mit dem Flp-Rekombinase-Expressionsplasmid pOG44 und einem modifizierten Plasmid, das zusätzlich das edg-1-Gen (Zugangsnummer NM_001400) enthält und als pcDNA5-FRT-TO_nFLAG_DEST-EDG-1 bezeichnet wird, mit einem Verhältnis von 9:1 kotransfiziert. Zum Erhalt des modifizierten pcDNA5-FRT-TO_nFLAG_DEST-Plasmids wurde das Invitrogen-Plasmid pcDNA5/FRT/TO (Invitrogen, Kat.-Nr. V6520-20) durch Insertierung einer Gateway-Kassette mit ein ccdB-Gen und ein Chloramphenicolresistenzgen flankierenden attR-Rekombinationsstellen (Gateway Conversion System, Invitrogen, Kat.-Nr. 11828-029) auf das Gateway^{®}-Kloniersystem (Invitrogen) angepasst. Außerdem wurde vor der 5'-att-Rekombinationsstelle ein FLAG-Tag-Epitop hinzugefügt, um eine rekombinante Expression von Proteinen mit N-terminalem FLAG-Tag zu ermöglichen.

Für die Transfektion einer Vertiefung wurden 1,08 µg pOG44 und 0,12 µg pcDNA5-FRT-TO_nFLAG_DEST-EDG-1 mit 100 µl serumfreiem Ham-F-12-Medium mit 6 µl FuGENE^{®}-6-Transfektionsreagens gemischt. Nach 20 min Inkubation wurde der Transfektionsreagens/DNA-Komplex tropfenweise auf den Zellen verteilt. Die Zellen wurden 24 h bei 37°C inkubiert. Dann wurden die Zellen aus drei Vertiefungen in eine T75-Flasche (Greiner Cellstar^{®}, Kat.-Nr. 658175) mit Ham-F-12-Medium mit 10% FCS, aber ohne Antibiotikum, überführt und noch 24 h inkubiert. 48 h nach der Transfektion wurde das Medium durch Selektionsmedium (Ham F-12 mit 10 % FCS und 300 µg/ml Hygromycin B (Invitrogen, Kat.-Nr. 10687-010)) ersetzt. Das Medium wurde alle 2 bis 3 Tage ausgetauscht, bis eine resistente Population von Zellen herangewachsen war. Zellen wurden mehrmals aufgeteilt und in eine neue Flasche ausgesät, so daß die Zellen nicht mehr als 25% Konfluenz erreichten. Nach 2 Wochen Selektion wurden die Zellen in T175-Flaschen (Greiner Cellstar^{®}, Kat.-Nr. 660175) überführt und für die Batchproduktion kultiviert. Die Zellen wurden durch kurze Behandlung (2 bis 5 min) mit Accutase (PAA, Kat.-Nr. L11-007) aus den Kulturflaschen geerntet, in Selektionsmedium (siehe oben) resuspendiert und 5 min bei 200 x g zentrifugiert. Die Zellen wurden in einer Mischung von 90% FCS und 10% Dimethylsulfoxid resuspendiert und in flüssigem Stickstoff gefroren gelagert.

### (b) Membranpräparat

Aus der obenbeschriebenen CHO-Flp-In-Zelllinie, die den humanen Edg-1-Rezeptor konstitutiv überexprimiert, wurde nach Standardmethoden ein Membranpräparat erhalten. Kurz gesagt, wurden die kryokonservierten Zellen in Kultur genommen und in T175-Zellkulturflaschen (Becton Dickinson, Kat.-Nr. 35 5001) bis zur Konfluenz angezogen. Die Zellkultur wurde durch Waschen mit calciumfreier phosphatgepufferter Kochsalzlösung (PBS; Gibco, Kat.-Nr. 14190) gestoppt, und die Zellen wurden mit einem Gummischaber in 4°C kaltem und calciumfreiem PBS mit einem Proteaseinhibitorcocktail (Complete Protease Inhibitor; Roche, Kat.-Nr. 1697498; 1 Tablette pro 50 ml) geerntet und danach bei 4°C 15 min bei 1100 x g zentrifugiert (Heraeus Minifuge T). Zur Zelllyse wurde das Pellett in 4°C kaltem hypotonischem Puffer aus 5 mM HEPES (Sigma-Aldrich, Kat.-Nr. H-0981), 1 mM EDTA (Dinatriumsalz; Merck, Kat.-Nr. 8418) mit Proteaseinhibitorcocktail (wie oben) resuspendiert, in dem die Zellen noch 15 min auf Eis gelagert wurden. Nach der Lyse wurden die Zellen bei 4°C 10 min bei 400 x g zentrifugiert (Heraeus Minifuge T). Das Pellet wurde in einem Dounce-Homogenisator auseinandergebrochen, mit dem Überstand der vorhergehenden Zentrifugation verdünnt und danach bei 4°C 10 min bei 500 x g zentrifugiert (Heraeus Minifuge T), um Nuklei und noch intakte Zellen von den hauptsächlich im Überstand vorliegenden Membranen abzutrennen. Der Überstand wurde dann in hypotonischem Puffer verdünnt und bei 4°C bei ungefähr 18600 x g 2 h zentrifugiert (Beckmann, Avanti J251). Danach wurde das Membranpellet in einem Lagerpuffer aus 20 mM HEPES; 150 mM NaCl (Merck, Kat.-Nr. 6400), 1 mM EDTA (wie oben) mit Proteaseinhibitorcocktail (wie oben) resuspendiert. Das Membranpräparat wurde aliquotiert und bei -80°C gelagert. Die Proteinkonzentration des Membranpräparats wurde in einer Probe mit Hilfe eines kommerziellen Proteinassays (Bio-Rad, DC Protein Assay, Kat.-Nr. 500-0113, 500-0114, 500-0115) bestimmt.

### (c) GTP-γ-S-Assay

Das in (b) erhaltene Edg-1-Membranpräparat wurde in einem kommerziell erhältlichen Szintillationsproximitätsassay-Kit (SPA-Kit) auf die Bindung am G-Protein-gekoppelten Rezeptor von Amersham Biosciences/GE Healthcare (Code RPNQ0210) eingesetzt, indem die ligandeninduzierte Bindung von ³⁵S-radiomarkiertem GTP-γ-S an die rezeptorhaltige Membran, die an Szintillationsperlen gebunden ist, die Emission von Licht induziert und die Quantifizierung der in-vitro-Wirkung der Edg-1-agonistischen Verbindung erlaubt. Der Assay wurde auf einer Platte mit 96 Vertiefungen weitgehend nach den Anweisungen des Herstellers durchgeführt. Vor dem Beginn der Experimente wurden Szintillationsperlen in einem Rekonstitutionspuffer aus Tris-HCl (pH 7,4) mit 0,1% (w/v) Natriumazid suspendiert und dann auf Eis mit Assaypuffer (aus 20 mM HEPES, 100 mM NaCl, 1 mM EDTA (wie oben), 1 mM Dithiothreitol (DTT), auf pH 7,4 eingestellt) auf eine Perlenendkonzentration von 30 mg/ml verdünnt.

Die Vertiefungen wurden mit 10 µl des angegebenen Assaypuffers, 10 µl 100 µM Guanosindiphosphat-Lösung (GDP-Lösung) und 10 µl einer Lösung der Testverbindung in Assaypuffer/Dimethylsulfoxid versetzt, was eine Endkonzentration der Testverbindung von 10 µM ergibt. Anstelle der Lösung der Testverbindung wurden für die hohen Kontrollen 10 µl einer Lösung von Sphingosin-1-phosphat (S1 P; Sigma, Kat.-Nr. S-9666), was eine S1 P-Endkonzentration von 10 µM ergab, und für die niedrigen Kontrollen 10 µl Assaypuffer in die jeweiligen Vertiefungen gegeben. Alle Vertiefungen enthielten äquivalente Mengen von Dimethylsulfoxid. Dann wurden in jede Vertiefung 10 µl einer [³⁵S]GTP-γ-S-Lösung (4 nM) und das in (b) erhaltene Edg-1-Membranpräparat (15 µg Membranprotein in 100 µl Assaypuffer) gegeben. Nach Inkubation der Platten bei Raumtemperatur über einen Zeitraum von 5 min wurden 50 µl der angegebenen Szintillationsperlensuspension (30 mg/ml) zugegeben. Nach einem weiteren Inkubationszeitraum von 45 min bei Raumtemperatur wurden die Platten 10 min bei 500 x g zentrifugiert. Die Quantifizierung der [³⁵S]GTP-γ-S-Bindung und somit der Rezeptoraktivierung wurde mit Hilfe eines Beta-Zählers (MicroBeta, Wallac) über einen Zeitraum von 1 min gemessen. Die Werte wurden durch Subtraktion der jeweiligen niedrigen Kontrolle hintergrundkorrigiert. Alle Messungen wurden dreifach durchgeführt. Die Rezeptoraktivierung durch die Testverbindung wird in % der jeweiligen hohen Kontrolle (10 µM S1 P; wird als 100% Aktivierung erachtet) ausgedrückt. Die mit der Beispielverbindung 1 bei 10 µM beobachtete Aktivierung betrug 86%.

Aus den Messdaten ist ersichtlich, dass die Verbindungen gut zur Wundheilung und insbesondere zur Behandlung von Wundheilungsstörungen von Diabetes Patienten geeignet sind.

## Patentansprüche

1. Verbindung der Formel I in einer beliebigen ihrer stereoisomeren Formen oder einer Mischung von stereoisomeren Formen in beliebigem Verhältnis oder physiologisch akzeptables Salz davon oder physiologisch akzeptables Solvat einer derartigen
Verbindung oder eines derartigen Salzes, 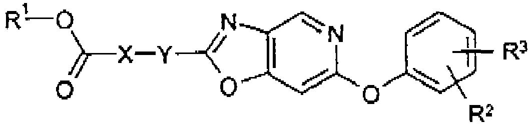 **dadurch gekennzeichnet, daß** darin bedeuten
X (C₁-C₆)-Alkandiyloxy, wobei das Sauerstoffatom der (C₁-C₆)-Alkandiyloxygruppe an die Gruppe Y gebunden ist;
Y Phenylen, wobei das Phenylen gegebenenfalls an einem oder mehreren Ringkohlenstoffatomen durch gleiche oder verschiedene Substituenten R⁵ substituiert ist;
R¹ Wasserstoff oder (C₁-C₄)-Alkyl;
R² und R³ unabhängig voneinander ausgewählt sind aus H, Halogen, Hydroxy, (C₁-C₄)-Alkyl-, (C₁-C₄)-Alkyloxy, (C₁-C₄)-Alkyl-S(O)ₘ-, Amino, Nitro, Cyano, Hydroxycarbonyl, (C₁-C₄)-Alkyloxycarbonyl, Aminocarbonyl und Aminosulfonyl, (C₃-C₇)-Cycloalkyl-C_{w}H_{2w}- und Oxy, wobei w aus 0, 1 und 2 ausgewählt ist;
R⁵ (C₁-C₄)-Alkyl,
m aus 0, 1 und 2 ausgewählt ist.

2. Verbindung der Formel I in einer beliebigen ihrer stereoisomeren Formen oder einer Mischung von stereoisomeren Formen in beliebigem Verhältnis oder physiologisch akzeptables Salz davon oder physiologisch akzeptables Solvat einer derartigen Verbindung oder eines derartigen Salzes gemäß Anspruch 1, **dadurch gekennzeichnet, daß** darin bedeuten
X -(CH₂)-O-, wobei -O- an die Gruppe Y gebunden ist;
Y Phenylen, wobei das Phenylen gegebenenfalls an einem oder mehreren Ringkohlenstoffatomen durch gleiche oder verschiedene Substituenten R⁵ substituiert ist;
R¹ Wasserstoff,;
R² und R³ unabhängig voneinander ausgewählt sind aus H, Halogen;
R⁵ (C₁-C₄)-Alkyl.

3. Pharmazeutische Zusammensetzung, enthaltend mindestens eine Verbindung der Formel I nach einem der Ansprüche 1 bis 2 oder ein physiologisch akzeptables Salz davon oder ein physiologisch akzeptables Solvat einer derartigen Verbindung oder eines derartigen Salzes und einen pharmazeutisch akzeptablen Träger.

4. Pharmazeutische Zusammensetzung, nach Anspruch 3, **dadurch gekennzeichnet, dass** es sich um eine Hydrogelzubereitung handelt.

5. Verbindung der Formel I nach einem der Ansprüche 1 bis 2 oder physiologisch akzeptables Salz davon oder physiologisch akzeptables Solvat einer derartigen Verbindung oder eines derartigen Salzes zur Verwendung als Arzneimittel.

6. Verbindung der Formel I nach einem der Ansprüche 1 bis 2 oder eines physiologisch akzeptablen Salzes davon oder eines physiologisch akzeptablen Solvats einer derartigen Verbindung oder eines derartigen Salzes zur Behandlung von Wundheilungsstörungen.

7. Verbindung der Formel I nach einem der Ansprüche 1 bis 2 oder eines physiologisch akzeptablen Salzes davon oder eines physiologisch akzeptablen Solvats einer derartigen Verbindung oder eines derartigen Salzes zur Wundheilung.

8. Verbindung der Formel I nach einem der Ansprüche 1 bis 2 oder eines physiologisch akzeptablen Salzes davon oder eines physiologisch akzeptablen Solvats einer derartigen Verbindung oder eines derartigen Salzes zur Wundheilung bei Diabetikern.

9. Verbindung der Formel I nach einem der Ansprüche 1 bis 2 oder eines physiologisch akzeptablen Salzes davon oder eines physiologisch akzeptablen Solvats einer derartigen Verbindung oder eines derartigen Salzes zur Behandlung von Herz-Kreislauf-Erkrankungen.

10. Verbindung der Formel I nach einem der Ansprüche 1 bis 2 oder eines physiologisch akzeptablen Salzes davon oder eines physiologisch akzeptablen Solvats einer derartigen Verbindung oder eines derartigen Salzes zur Cardioprotektion.

11. Verbindung der Formel I nach einem der Ansprüche 1 bis 2 oder eines physiologisch akzeptablen Salzes davon oder eines physiologisch akzeptablen Solvats einer derartigen Verbindung oder eines derartigen Salzes zur Behandlung des Diabetischen Fußsyndroms.

## Claims

1. Compound of the formula I, in any of its stereoisomeric forms, or a mixture of stereoisomeric forms in any ratio, or a physiologically acceptable salt thereof, or a physiologically acceptable solvate of such a compound or such a salt, 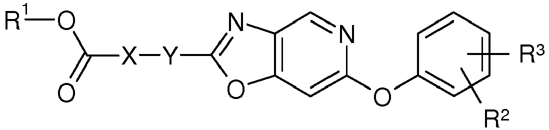 **characterized in that**
X is (C₁-C₆)-alkanediyloxy, where the oxygen atom of the (C₁-C₆)-alkanediyloxy group is attached to the group Y;
Y is phenylene, where the phenylene is optionally substituted at one or more ring carbon atoms by identical or different substituents R⁵;
R¹ is hydrogen or (C₁-C₄)-alkyl;
R² and R³ independently of one another are selected from the group consisting of H, halogen, hydroxyl, (C₁-C₄)-alkyl-, (C₁-C₄)-alkyloxy, (C₁-C₄)-alkyl-S(O)ₘ-, amino, nitro, cyano, hydroxycarbonyl, (C₁-C₄)-alkyloxycarbonyl, aminocarbonyl and aminosulfonyl, (C₃-C₇)-cycloalkyl-C_{w}H_{2w}- and oxy, where w is selected from the group consisting of 0, 1 and 2;
R⁵ is (C₁-C₄)-alkyl;
m is selected from the group consisting of 0, 1 and 2.

2. Compound of the formula I, in any of its stereoisomeric forms, or a mixture of stereoisomeric forms in any ratio, or a physiologically acceptable salt thereof, or a physiologically acceptable solvate of such a compound or such a salt according to Claim 1, **characterized in that**
X is -(CH₂)-O-, where -O- is attached to the group Y;
Y is phenylene, where the phenylene is optionally substituted at one or more ring carbon atoms by identical or different substituents R⁵;
R¹ is hydrogen;
R² and R³ independently of one another are selected from the group consisting of H, halogen;
R⁵ is (C₁-C₄)-alkyl.

3. Pharmaceutical composition, comprising at least one compound of the formula I according to either of Claims 1 and 2 or a physiologically acceptable salt thereof, or a physiologically acceptable solvate of such a compound or such a salt, and a pharmaceutically acceptable carrier.

4. Pharmaceutical composition according to Claim 3, **characterized in that** the pharmaceutical composition is a hydrogel preparation.

5. Compound of the formula I according to either of Claims 1 and 2 or a physiologically acceptable salt thereof, or a physiologically acceptable solvate of such a compound or such a salt, for use as a medicament.

6. Compound of the formula I according to either of Claims 1 and 2 or a physiologically acceptable salt thereof, or a physiologically acceptable solvate of such a compound or such a salt, for the treatment of wound healing disorders.

7. Compound of the formula I according to either of Claims 1 and 2 or a physiologically acceptable salt thereof, or a physiologically acceptable solvate of such a compound or such a salt, for wound healing.

8. Compound of the formula I according to either of Claims 1 and 2 or a physiologically acceptable salt thereof, or a physiologically acceptable solvate of such a compound or such a salt, for wound healing in diabetics.

9. Compound of the formula I according to either of Claims 1 and 2 or a physiologically acceptable salt thereof, or a physiologically acceptable solvate of such a compound or such a salt, for the treatment of cardiovascular disorders.

10. Compound of the formula I according to either of Claims 1 and 2 or a physiologically acceptable salt thereof, or a physiologically acceptable solvate of such a compound or such a salt, for cardioprotection.

11. Compound of the formula I according to either of Claims 1 and 2 or a physiologically acceptable salt thereof, or a physiologically acceptable solvate of such a compound or such a salt, for the treatment of diabetic foot syndrome.

## Revendications

1. Composé de formule I sous l'une quelconque de ses formes stéréoisomères ou sous la forme d'un mélange de formes stéréoisomères en un rapport quelconque ou sel physiologiquement acceptable de celui-ci ou solvate physiologiquement acceptable d'un tel composé ou d'un tel sel, 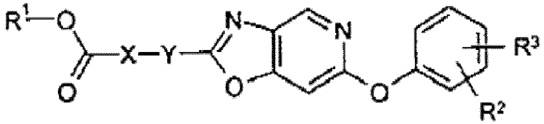 **caractérisé en ce que**, dans celui-ci,
X signifie alcanediyloxy en (C₁-C₆), l'atome d'oxygène du groupe alcanediyloxy en (C₁-C₆) étant relié au groupe Y,
Y signifie phénylène, le phénylène étant éventuellement substitué sur un ou plusieurs atomes de carbone de cycle par des substituants R⁵ identiques ou différents ;
R¹ signifie hydrogène ou alkyle en (C₁-C₄) ;
R² et R³ sont choisis indépendamment l'un de l'autre parmi H, halogène, hydroxy, alkyle en (C₁-C₄), alkyloxy en (C₁-C₄), alkyle en (C₁-C₄)-S(O)ₘ-, amino, nitro, cyano, hydroxycarbonyle, alkyloxycarbonyle en (C₁-C₄), aminocarbonyle et aminosulfonyle, cycloalkyle en (C₃-C₇)-C_{w}H_{2w}- et oxy, w étant choisi parmi 0, 1 et 2 ;
R⁵ signifie alkyle en (C₁-C₄) ;
m est choisi parmi 0, 1 et 2.

2. Composé de formule I sous l'une quelconque de ses formes stéréoisomères ou sous la forme d'un mélange de formes stéréoisomères en un rapport quelconque ou sel physiologiquement acceptable de celui-ci ou solvate physiologiquement acceptable d'un tel composé ou d'un tel sel selon la revendication 1, **caractérisé en ce que**, dans celui-ci,
X signifie -(CH₂)-O-, -O- étant relié au groupe Y ;
Y signifie phénylène, le phénylène étant éventuellement substitué sur un ou plusieurs atomes de carbone de cycle par des substituants R⁵ identiques ou différents ;
R¹ signifie hydrogène ;
R² et R³ sont choisis indépendamment l'un de l'autre parmi H, halogène ;
R⁵ signifie alkyle en (C₁-C₄).

3. Composition pharmaceutique, contenant au moins un composé de formule I selon l'une quelconque des revendications 1 à 2 ou un sel physiologiquement acceptable de celui-ci ou un solvate physiologiquement acceptable d'un tel composé ou d'un tel sel, et un véhicule pharmaceutiquement acceptable.

4. Composition pharmaceutique selon la revendication 3, **caractérisée en ce qu'**il s'agit d'une préparation d'hydrogel.

5. Composé de formule I selon l'une quelconque des revendications 1 à 2, ou sel physiologiquement acceptable de celui-ci ou solvate physiologiquement acceptable d'un tel composé ou d'un tel sel, destiné à une utilisation en tant que médicament.

6. Composé de formule I selon l'une quelconque des revendications 1 à 2, ou sel physiologiquement acceptable de celui-ci ou solvate physiologiquement acceptable d'un tel composé ou d'un tel sel, pour le traitement de troubles de la cicatrisation.

7. Composé de formule I selon l'une quelconque des revendications 1 à 2, ou sel physiologiquement acceptable de celui-ci ou solvate physiologiquement acceptable d'un tel composé ou d'un tel sel, pour la cicatrisation.

8. Composé de formule I selon l'une quelconque des revendications 1 à 2, ou sel physiologiquement acceptable de celui-ci ou solvate physiologiquement acceptable d'un tel composé ou d'un tel sel, pour la cicatrisation chez les diabétiques.

9. Composé de formule I selon l'une quelconque des revendications 1 à 2, ou sel physiologiquement acceptable de celui-ci ou solvate physiologiquement acceptable d'un tel composé ou d'un tel sel, pour le traitement de maladies cardio-vasculaires.

10. Composé de formule I selon l'une quelconque des revendications 1 à 2, ou sel physiologiquement acceptable de celui-ci ou solvate physiologiquement acceptable d'un tel composé ou d'un tel sel, pour la cardioprotection.

11. Composé de formule I selon l'une quelconque des revendications 1 à 2, ou sel physiologiquement acceptable de celui-ci ou solvate physiologiquement acceptable d'un tel composé ou d'un tel sel, pour le traitement du syndrome du pied diabétique.
